# FASCICULE DE BREVET EUROPEEN

(11) **EP 4 028 021 B1**
(45) Date de publication et mention de la délivrance du brevet: **24.01.2024**
(21) Numéro de dépôt: 20765054.0
(22) Date de dépôt: 08.09.2020
(51) Int. Cl.: A61K 31/706, A61K 31/7084, A61K 45/06, A61P 29/00, A61P 29/02

(54) **UTILISATION DE NMN POUR LA PRÉVENTION ET/OU LE TRAITEMENT DE LA DOULEUR ET COMPOSITIONS CORRESPONDANTES**
VERWENDUNG VON NMN ZUR PRÄVENTION UND/ODER BEHANDLUNG VON SCHMERZEN UND ENTSPRECHENDE ZUSAMMENSETZUNGEN
USE OF NMN FOR THE PREVENTION AND/OR TREATMENT OF PAIN, AND CORRESPONDING COMPOSITIONS

(30) Priorité: 09.09.2019 FR 1909896; 20.12.2019 EP 19218817
(43) Date de publication de la demande: 20.07.2022
(73) Titulaire: Nuvamid SA, 1066 Epalinges (CH)
(72) Inventeur: BERMOND, Guillaume, 1066 Epalinges (CH); GARÇON, Laurent, 1066 Epalinges (CH)
(74) Mandataire: August Debouzy
(86) Numéro de dépôt international: PCT/EP2020/075085
(87) Numéro de publication internationale: WO 2021/048129

(56) Documents cités:
- WO-A1-2016/176437
- WO-A1-2017/096246
- WO-A1-2020/081923
- CN-A- 107 233 352
- CN-A- 109 125 338
- CHANDRASEKARAN K ET AL: "A nicotinamide adenine nucleotide (NAD+) precursor is a potential therapy for diabetic neuropathy", 2016, JOURNAL OF NEUROMUSCULAR DISEASES 2016 IOS PRESS NLD, VOL. 3, NR. SUPPLEMENT 1, PAGE(S) S86 CONF 20160705 TO 20160709 TORONTO, ON- 14TH INTERNATIONAL CONGRESS ON NEUROMUSCULAR DISEASES, ICNMD 2016, XP002798965, ISSN: 2214-3602 le document en entier
- MARTA V. HAMITY ET AL: "Nicotinamide riboside, a form of vitamin B3 and NAD+ precursor, relieves the nociceptive and aversive dimensions of paclitaxel-induced peripheral neuropathy in female rats", PAIN, vol. 158, no. 5, 30 janvier 2017 (2017-01-30), pages 962-972, XP055767044, NL ISSN: 0304-3959, DOI: 10.1097/j.pain.0000000000000862
- DATABASE EMBASE [Online] ELSEVIER SCIENCE PUBLISHERS, AMSTERDAM, NL; 1 octobre 2017 (2017-10-01), KUMAR P H ET AL: "Nicotinamide riboside is a potential therapy for diabetic neuropathy", XP055767069, Database accession no. EMB-619084706 & KUMAR P H ET AL: "Nicotinamide riboside is a potential therapy for diabetic neuropathy", ANNALS OF NEUROLOGY 20171001 JOHN WILEY AND SONS INC. NLD, vol. 82, no. Supplement 21, 1 octobre 2017 (2017-10-01), pages S106 CONF 20171015 to 20171017 San Diego, CA-142nd Ann, ISSN: 1531-8249
- MIGAUD ET AL: "Probing Aplysia californica Adenosine 5'-Diphosphate Ribosyl Cyclase for Substrate Binding Requirements: Design of Potent Inhibitors", BIOCHEMISTRY,, vol. 38, no. 28, 1 janvier 1999 (1999-01-01), pages 9105-9114, XP002191910, ISSN: 0006-2960, DOI: 10.1021/BI9903392
- CHRISTOPH WOENCKHAUS ET AL: "Eigenschaften des Coenzymanalogen Bis-nicotinamid-dinucleotid [Properties of the coenzyme analog bisnicotinamide dinucleotide]", HOPPE-SEYLER'S ZEITSCHRIFT FUER PHYSIOLOGISCHE CHEMIE, WALTER DE GRUYTER, BERLIN, DE, vol. 354, no. 1, 1 janvier 1973 (1973-01-01), pages 53-59, XP009521164, ISSN: 0018-4888, DOI: 10.1515/BCHM2.1973.354.1.53 [extrait le 2009-10-15]

## Description

### DOMAINE DE L'INVENTION

La présente invention porte sur l'utilisation de nicotinamide mononucléotide (NMN), un de ses dérivés pharmaceutiquement acceptables ou un de ses sels pharmaceutiquement acceptables, pour le traitement et/ou la prévention de la douleur, à savoir la douleur nociceptive viscérale. La présente invention porte également sur des compositions comprenant du NMN, un de ses dérivés pharmaceutiquement acceptables ou un de ses sels pharmaceutiquement acceptable pour le traitement et/ou la prévention de la douleur nociceptive viscérale.

### ARRIERE-PLAN TECHNIQUE

La douleur peut être à la fois un symptôme et une maladie. La douleur est définie par le comité IASP (pour « International Association for the Study of Pain » en anglais) comme « *une expérience sensorielle et émotionnelle déplaisante associée à une lésion tissulaire réelle ou potentielle, ou décrite en termes d'une telle lésion* » (voir la définition au lien suivant selon la mise à jour du 14 décembre 2017: https://www.iasp-pain.org/Education/Content.aspx?ItemNumber=1698&navItemNumber=576#Pain ). La douleur est une information traitée par le système nerveux.

Le système nerveux des mammifères comme les humains comprend deux parties principales :
- le système nerveux central constitué par l'encéphale, le tronc cérébral, le cervelet et la moelle épinière. Le rôle du système nerveux central est de recevoir, d'enregistrer et d'interpréter les signaux qui viennent du système nerveux périphérique ; et
- le système nerveux périphérique constitué par les nerfs crâniens et spinaux, rattachés au système nerveux central, et leurs terminaisons. Le rôle du système nerveux périphérique est de (i) transmettre les informations reçues par les récepteurs périphériques de la sensibilité et de la douleur au système nerveux central et (ii) transmettre les ordres émis par le système nerveux central aux muscles notamment.

La douleur est un phénomène complexe et de ce fait, il existe différentes manières de la catégoriser. On distingue notamment trois grandes catégories de douleurs : la douleur nociceptive, la douleur neuropathique et la douleur centralisée.

La douleur nociceptive ou périphérique est causée par l'activation des nocicepteurs. Les nocicepteurs sont des récepteurs situés à l'extrémité des fibres nerveuses. Dans le cas de la douleur nociceptive, le système nerveux n'est pas atteint. Différents stimuli peuvent être à l'origine de la douleur nociceptive tels qu'un stimulus mécanique, un stimulus thermique, un stimulus chimique, une maladie inflammatoire ou une maladie infectieuse. On peut citer à titre d'exemple de douleur nociceptive la douleur due à une brûlure de la peau.

La douleur neuropathique est causée par des lésions au niveau des nerfs du système nerveux périphérique. On peut citer à titre d'exemple la douleur neuropathique diabétique.

La douleur centralisée est due à une perturbation du traitement de la douleur par le système nerveux central. On peut citer à titre d'exemple la fibromyalgie ou la douleur du membre fantôme.

En revanche, la migraine constitue une catégorie de douleur distincte de ces trois catégories et constitue, pour le moment, une catégorie distincte.

Il convient en outre de préciser que ces catégories de douleurs ne sont pas exclusives l'une de l'autre et qu'un patient peut ressentir différents types de douleurs simultanément. En effet, en cas de blessure importante, d'un cancer ou d'une maladie infectieuse, la lésion peut s'étendre à la fois aux tissus et aux nerfs présents dans l'organe.

La douleur nociceptive répond en général facilement aux traitements antalgiques conventionnels. L'Organisation Mondiale de la Santé classe les antalgiques en trois catégories en fonction de leur puissance d'action. On qualifie d'antalgique une substance permettant de diminuer la douleur.

Les substances antalgiques de niveau I sont destinées aux douleurs légères à modérées et comprennent l'aspirine, le paracétamol et les anti-inflammatoires non stéroïdiens (AINS) tels que l'ibuprofène, le kétoprofène, le naproxène, l'alminoprofène l'acéclofénac, l'acide méfénamique, l'acide niflumique, l'acide tiaprofénique, le célécoxib, le dexkétoprofène, le diclofénac, l'étodolac, l'étoricoxib, le fénoprofène, le flurbiprofène, l'indométacine, le méloxicam, le nabumétone, le piroxicam, le sulindac et le ténoxicam. Les AINS sont désignés ainsi pour les distinguer des anti-inflammatoires stéroïdiens ou corticoïdes, dérivés du cortisol, l'une des hormones libérées lors de la réaction de stress. On peut citer à titre d'exemple de corticoïde la bétaméthasone, la ciprofloxacine, le cortivazol, la dexaméthasone, la fludrocortisone, le methylprednisolone, la prednisolone et la triamcinolone.

Les substances antalgiques de niveau II sont destinées aux douleurs modérées ou sévères ou aux douleurs insuffisamment soulagées par les antalgiques de niveau I et comprennent la codéine, la dihydrocodéine et le tramadol, seuls ou associés à l'aspirine ou au paracétamol.

Les substances antalgiques de niveau III sont destinées aux douleurs intenses rebelles aux autres antalgiques et comprennent la morphine et les autres dérivés de l'opium tels que la buprénorphine, le fentanyl, l'hydromorphone, la nalbuphine, l'oxycodone et la péthidine.

Cependant, aucun de ces traitements n'est exempt d'effet secondaire. Par exemple l'utilisation d'AINS peut causer différents effets secondaires tels que des hémorragies, de l'asthme, des troubles rénaux et plus fréquemment des troubles gastriques et des ulcères. Le paracétamol peut engendrer une toxicité hépatique. L'aspirine fluidifie le sang et agresse l'estomac. Les corticoïdes engendrent une prise de poids, un affaiblissement des défenses immunitaires, une fragilisation des os et une cortico-dépendance entrainant une réduction de leur efficacité. La codéine, la dihydrocodéine et le tramadol induisent, parmi les effets secondaires les plus fréquents, des nausées, vomissements, constipation, somnolence et une dépendance. Quant à la morphine et les dérivés de l'opium, ils induisent d'importants effets secondaires et notamment un risque élevé de dépendance physique et psychologique. Par ailleurs, un surdosage en morphine bloque les muscles respiratoires et peut se révéler létal.

Enfin, certains patients peuvent développer des allergies aux antalgiques conventionnels.

Il existe donc un besoin de développer de nouvelles compositions pour le traitement et/ou la prévention de la douleur qui réduisent les inconvénients des antalgiques de l'art antérieur.

Le document WO 2016/176437 décrit l'utilisation de la nicotinamide mononucléotide (NMN) dans la prévention ou le traitement de la douleur, en particulier neuropathique.

### RESUME DE L'INVENTION

Ces objectifs sont atteints grâce au nicotinamide mononucléotide (NMN) et des compositions le comprenant pour leur utilisation dans la prévention et/ou le traitement de la douleur.

La présente invention a pour objet le nicotinamide mononucléotide (NMN), un de ses dérivés pharmaceutiquement acceptables ou un de ses sels pharmaceutiquement acceptables, tel que défini dans la revendication 1, pour son utilisation dans la prévention et/ou le traitement de la la douleur nociceptive viscérale qui n'est pas une douleur neuropathique.

Avantageusement, le NMN est utilisé dans une quantité comprise entre 0,01 mg/kg/jour et 1000 mg/kg/jour, de préférence entre 1 mg/kg/jour et 100 mg/kg/jour, de manière davantage préférée entre 5 mg/kg/jour et 50 mg/kg/jour, de manière encore plus préférée entre 10 et 20 mg/kg/jour.

Dans un mode de réalisation, le dérivé du NMN peut être choisi parmi l'alpha nicotinamide mononucléotide (α-NMN), le dihydronicotinamide mononucléotide (noté NMN-H), le composé de formule (I): 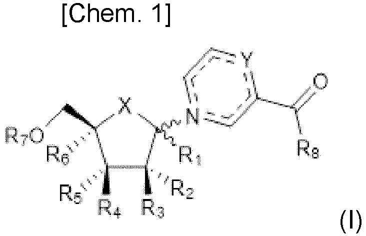 ou un de ses stéréoisomères, un de ses sels, un de ses hydrates, un de ses solvates ou un de ses cristaux pharmaceutiquement acceptable de celui-ci, dans lequel :
- X est choisi parmi O, CH₂, S, Se, CHF, CF₂ et C=CH₂;
- R₁ est choisi parmi H, azido, cyano, alkyle en (C₁-C₈), thio-alkyle en (C₁-C₈), hétéroalkyle en (C₁-C₈) et OR ; dans lequel R est choisi parmi H et alkyle en (C₁-C₈) ;
- R₂, R₃, R₄ et R₅ sont choisis indépendamment l'un de l'autre parmi H, halogène, azido, cyano, hydroxyle, alkyle en (C₁-C₁₂), thio-alkyle en (C₁-C₁₂), hétéroalkyle en (C₁-C₁₂), haloalkyle en (C₁-C₁₂) et OR ; dans lequel R est choisi parmi H, alkyle en (C₁-C₁₂), C(O)(C₁-C₁₂)alkyle, C(O)NH(C₁-C₁₂)alkyle, C(O)O(C₁-C₁₂)alkyle, C(O)aryle, C(O)(C₁-C₁₂)alkyle aryle, C(O)NH(C₁-C₁₂)alkyle aryle, C(O)O(C₁-C₁₂)alkyle aryle et C(O)CHR_{AA}NH₂ ; dans lequel R_{AA} est une chaîne latérale choisie parmi un acide aminé protéinogène ;
- R₆ est choisi parmi H, azido, cyano, (C₁-C₈) alkyle, (C₁-C₈) thio-alkyle, (C₁-C₈) hétéroalkyle et OR ; dans lequel R est choisi parmi H et (C₁-C₈) alkyle;
- R₇ est choisi parmi H, P(O)R₉R₁₀ et P(S)R₉R₁₀ ; dans lequel
- R₉ et R₁₀ sont choisis indépendamment l'un de l'autre parmi OH, OR₁₁, NHR₁₃, NR₁₃R₁₄, un alkyle en (C₁-C₈), un alcényle en (C₂-C₈), un alcynyl en (C₂-C₈), un cycloalkyl en (C₃-C₁₀), un aryle en (C₅-C₁₂), (C₁-C₈)alkyle aryle, (C₁-C₈)aryle alkyle, (C₁-C₈) heteroalkyle, (C₁-C₈) heterocycloalkyle, un heteroaryle et NHCHR_{A}R_{A'}C(O)R₁₂ ; dans lequel :
- R₁₁ est choisi parmi un groupe (C₁-C₁₀) alkyle, (C₃-C₁₀) cycloalkyle, (C₅-C₁₈) aryle, (C₁-C₁₀) alkylaryle, (C₅-C₁₂) aryle substitué, (C₁-C₁₀) heteroalkyle, (C₃₋C₁₀) heterocycloalkyle, (C₁-C₁₀ haloalkyle, un heteroaryle, -(CH₂)ₙC(O)(C₁-C₁₅)alkyle, - (CH₂)ₙOC(O)(C₁-C₁₅)alkyle, -(CH₂)ₙOC(O)O(C₁-C₁₅)alkyle, -(CH₂)ₙSC(O)(C₁-C₁₅)alkyle, -(CH₂)ₙC(O)O(C₁-C₁₅)alkyle and -(CH₂)ₙC(O)O(C₁-C₁₅)alkyle aryle; dans lesquels n est un entier choisi de 1 à 8; P(O)(OH)OP(O)(OH)₂; halogène, nitro, cyano, C₁-C₆ alkoxy, C₁-C₆ haloalkoxy, -N(R₁₁ₐ)₂, C₁-C₆ acylamino, -COR_{11b}, -O COR_{11b} ; NHSO₂(C₁-C₆ alkyl), - SO₂N(R₁₁ₐ)₂ SO₂ dans lequel chacun de R₁₁ₐ est indépendamment choisi parmi H et un (C₁-C₆) alkyle et R_{11b} est indépendamment choisi parmi OH, C₁-C₆ alkoxy, NH₂, NH(C₁-C₆ alkyle) ou N(C₁-C₆ alkyle)₂ ;
- R₁₂ est choisi parmi H, C₁₋C₁₀ alkyle, C₂-C₈ alcényle, C₂-C₈ alcynyle, C₁₋C₁₀ haloalkyle, C₃₋C₁₀ cycloalkyle, C₃₋C₁₀ heterocycloalkyle, C₅-C₁₈ aryle, C₁-C₄ alkylaryle and C₅-C₁₂ heteroaryle; dans lesquels lesdits groupes aryle ou heteroaryle sont optionnellement substitué parmi un ou deux groupes choisi parmi un halogène, trifluoromethyl, C₁-C₆ alkyle, C₁-C₆ alkoxy et cyano; et
- R_{A} et R_{A'} sont indépendamment choisi parmi H, un (C₁₋C₁₀) alkyle, (C₂-C₁₀) alcényle, (C₂-C₁₀) alcynyle, (C₃₋C₁₀) cycloalkyle, (C₁-C₁₀) thio-alkyle, (C₁₋C₁₀) hydroxylalkyle, (C₁-C₁₀) alkylaryle and (C₅-C₁₂) aryle, (C₃₋C₁₀) heterocycloalkyle, un heteroaryle, -(CH₂)₃NHC(=NH)NH₂, (1H-indol-3-yl)methyl, (1H-imidazol-4-yl)methyl et une chaine latérale choisie parmi un acide aminé protéinogène ou un acide aminé non protéinogène; dans lesquels lesdits groupes aryle sont optionnellement substitué avec un groupe choisi parmi un hydroxyle, (C₁₋C₁₀) alkyl, (C₁₋C₆) alkoxy, un halogène, un nitro et un cyano; ou
- R₉ et R₁₀ forment ensemble avec les atomes de phosphore auxquels ils sont attaché un cycle à 6 membres dans lequel -R₉-R₁₀- représente -CH₂-CH₂-CHR-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un C₁-C₆ alkyle, un (C₁-C₆) alkoxy et cyano; ou
   R₉ et R₁₀ forment ensemble avec les atomes de phosphore auxquels ils sont attaché un cycle à 6 membres dans lequel -R₉-R₁₀- représente -O-CH₂-CH₂-CHR-O-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un (C₁-C₆) alkyle, un (C₁-C₆) alkoxy et cyano;
- R₈ est choisi parmi H, OR, NHR₁₃, NR₁₃R₁₄, NH-NHR₁₃, SH, CN, N₃ et halogène ; dans lequel R₁₃ et R₁₄ sont choisis indépendamment l'un de l'autre parmi H, (C₁-C₈) alkyle, (C₁-C₈) alkyle aryle, et -CR_{B}R_{C}-C(O)-OR_{D} dans lequel R_{B} et Rc sont indépendamment un atome d'hydrogène, un (C₁-C₆) alkyle, un (C₁-C₆) alkoxy, benzyle, indolyle ou imidazolyle, où le (C₁-C₆) alkyle et le (C₁-C₆) alkoxy peuvent être éventuellement et indépendamment l'un de l'autre substitué par un ou plusieurs des groupes halogène, amino, amido, guanidyle, hydroxyle, thiol ou carboxyle, et le groupe benzyle est éventuellement substitué par un ou plusieurs des groupes halogène ou hydroxyle, ou R_{B} et Reforment ensemble avec l'atome de carbone auquel ils sont attachés un groupe cycloalkyle en C₃-C₆ éventuellement substitué par un ou plusieurs halogènes, amino, amido, guanidyle, hydroxyle, thiol et carboxyle et R_{D} est un hydrogène, un (C₁-C₆) alkyle, un (C₂-C₆) alcényle, un (C₂-C₆) alcynyle ou un (C₃-C₆) cycloalkyle ;
- Y est choisi parmi CH, CH₂, C(CH₃)₂ et CCH₃ ;
- 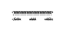 représente une simple ou une double liaison selon Y ; et
- 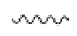 représente l'anomère alpha ou bêta selon la position de R₁
   ou un de ses stéréoisomères, un de ses sels, un de ses hydrates, un de ses solvates ou un de ses cristaux
      ou
      le composé de formule (II) : 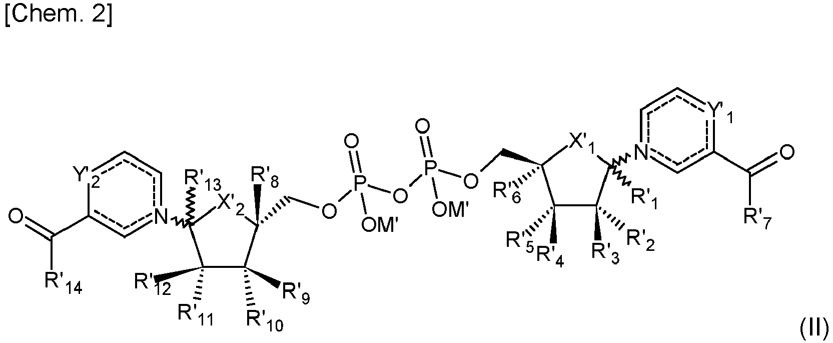
   ou un de ses stéréoisomères, un de ses sels, un de ses hydrates, un de ses solvates ou un de ses cristaux, dans lequel
      - X'₁ et X'₂ sont indépendamment choisis parmi O, CH₂, S, Se, CHF, CF₂, et C=CH₂ ;
      - R'₁ et R'13 sont indépendamment choisis parmi H, azido, cyano, un alkyl en C1-C8, un thio-alkyl en C1-C8, un hétéroalkyl en C1-C8, et OR, dans lequel R est choisi parmi H et un alkyl en C1-C8,
      - R'₂, R'₃, R'₄, R'₅, R'₉, R'₁₀, R'₁₁, R'₁₂ sont indépendamment choisis parmi H, un halogène, un azido, un cyano, un hydroxyl, un alkyl en C₁-C₁₂, un thioalkyl en C₁-C₁₂, un hétéro-alkyl en C₁-C₁₂, un haloalkyl en C₁-C₁₂ et OR, dans lequel R peut être choisi parmi H, un alkyl en C₁-C₁₂, un C(O)( C₁-C₁₂) alkyle, un C(O)NH(C₁-C₁₂) alkyle, un C(O)O(C₁-C₁₂) alkyle, un C(O) aryle, un C(O)(C₁-C₁₂) aryle, un C(O)NH(C₁-C₁₂) alkyle aryle, un C(O)O(C₁-C₁₂) alkyle aryle ou un groupe C(O)CHR_{AA}NH₂ dans lequel R_{AA} est une chaine latérale choisi parmi un acide aminé protéinogène ;
      - R'₆ et R'₈ sont indépendamment choisis parmi H, un azido, un cyano, un alkyl en C₁-C₈ et OR, dans lequel R est choisi parmi H et un alkyl en C₁-C₈ ;
      - R'₇ et R'₁₄ sont indépendamment choisis parmi H, OR, NHR, NRR', NH-NHR, SH, CN, N₃ et un halogène, dans lequel R et R' sont indépendamment choisit parmi H et un (C₁-C₈) alkyle aryle ;
      - Y'₁ et Y'₂ sont indépendamment choisis parmi CH, CH₂, C(CH₃)₂ ou CCH₃ ;
      - M' est choisi parmi H ou un contre-ion adapté ;
      - 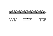 représente une liaison simple ou double en fonction de Y'₁ et Y'₂ ; et
      - 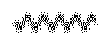 représente un anomère alpha ou bêta en fonction de la position de R'₁ et R'₁₃;
   et leurs combinaisons.
A noter que seuls les composés de formule (I) ou (II) revendiqués font parties de la présente invention.

Dans un premier mode non-revendiqué de réalisation, le dérivé pharmaceutiquement acceptable est le composé de formule (I).

Dans une variante du premier mode de réalisation, X représente un oxygène.

Dans une variante du premier mode de réalisation, R₁ et R₆ représentent chacun indépendamment l'un de l'autre un hydrogène.

Dans une variante du premier mode de réalisation, R₂, R₃, R₄ et R₅ représentent chacun indépendamment l'un de l'autre un hydrogène ou un OH.

Dans une variante du premier mode de réalisation, Y représente un CH.

Dans une variante du premier mode de réalisation, Y représente un CH₂.

Dans une variante du premier mode de réalisation, R₇ représente un hydrogène.

Dans une variante du premier mode de réalisation, R₇ représente P(O)(OH)₂.

Dans une variante du premier mode de réalisation,
X représente un oxygène ; et/ou
R₁ et R₆ représente chacun indépendamment un hydrogène ; et/ou
R₂, R₃, R₄ et R₅ représente chacun indépendamment un hydrogène ou R₂, R₃, R₄ et R₅ représente indépendamment OH ; et/ou
Y représente un CH ou un CH₂ ; et/ou
R₇ représente P(O)R₉R₁₀, dans lequel R₉ et R₁₀ sont indépendamment choisis parmi OH, OR₁₁, NHR₁₃, NR₁₃R₁₄, C₁-C₈ alkyle, C₂-C₈ alcényle, C₂-C₈ alcynyle, C₃₋C₁₀ cycloalkyle, C₅-C₁₂ aryle, C₁-C₈ aryle alkyle, C₁-C₈ alkyle aryle, C₁-C₈ heteroalkyle, C₁-C₈ heterocycloalkyle, heteroaryle et NHCR_{A}R_{A'}C(O)R₁₂.

Dans une variante particulièrement préférée du premier mode de réalisation, le composé de l'invention est choisi parmi les composés de formule I-A à I-H :

**[Tableau 1]**

| Composés n° (anomères) | Structure |
|---|---|
| I-A (beta) | 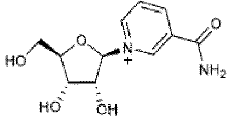 |
| I-B (alpha) | 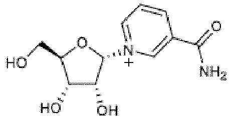 |
| I-C (beta) | 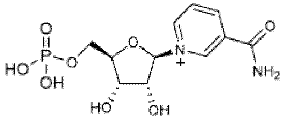 |
| I-D (alpha) | 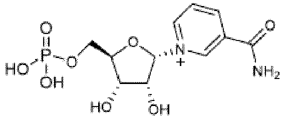 |
| I-E (beta) | 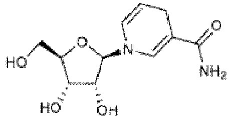 |
| I-F (alpha) | 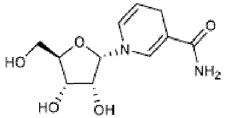 |
| I-G (beta) | 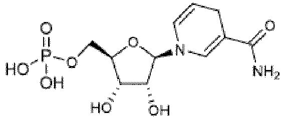 |
| I-H (alpha) | 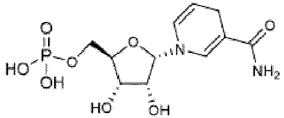 |

| | |
|---|---|
| Seuls les composés I-C, I-D, I-G et I-H font parties de l'invention. | |

Dans un deuxième mode non-revendiqué de réalisation, le dérivé pharmaceutiquement acceptable est le composé de formule (II).

Dans une variante du deuxième mode de réalisation, X'1 et X'2 représentent chacun indépendamment un oxygène.

Dans une variante du deuxième mode de réalisation, R'7 et R'14 représentent chacun indépendamment un NH₂.

Dans une variante du deuxième mode de réalisation, R'1 et/ou R'13 représentent chacun indépendamment un hydrogène.

Dans une variante du deuxième mode de réalisation, R'6 et/ou R'8 représentent chacun indépendamment un hydrogène.

Dans une variante du deuxième mode de réalisation, R'2, R'3, R'4, R'5, R'9, R'10, R'11 et R'12 représentent chacun indépendamment un hydrogène.

Dans une variante du deuxième mode de réalisation, R'2, R'3, R'4, R'5, R'9, R'10, R'11 et R'12 représentent chacun indépendamment un OH.

Dans une variante du deuxième mode de réalisation, Y'1 et Y'2 représentent chacun indépendamment un CH.

Dans une variante du deuxième mode de réalisation, Y'1 et Y'2 représentent chacun indépendamment un CH2.

Le composé selon l'invention est choisi parmi les composés de formule II-A à II-F :

**[Tableau 2]**

| Composé n° (anomère) | Structure |
|---|---|
| II-A (bêta, bêta) | 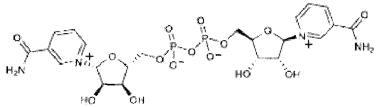 |
| II-8 (bêta, alpha) | 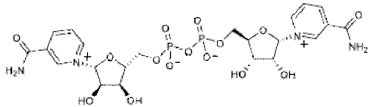 |
| II-C (alpha, alpha) | 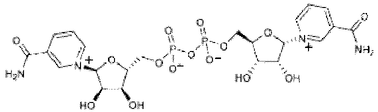 |
| II-Q (bêta, bêta) | 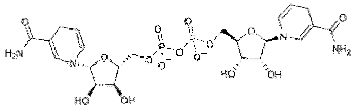 |
| II-E (bêta, alpha) | 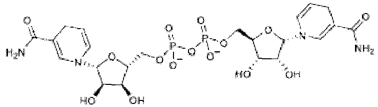 |
| II-F (alpha, alpha) | 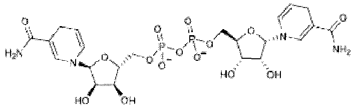 |

Dans une variante préférée de l'invention, le dérivé pharmaceutiquement acceptable est l'alpha-NMN de formule :

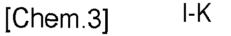

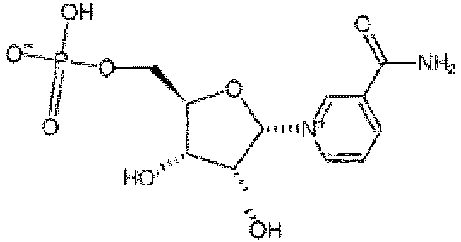

Dans un quatrième mode de réalisation préféré, le dérivé pharmaceutiquement acceptable est le NMN-H :

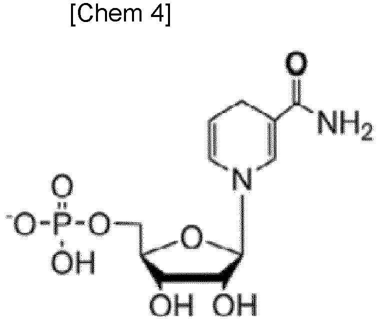

Avantageusement, le précurseur pharmaceutiquement acceptable non-revendiqué est le nicotinamide riboside (noté NR) : 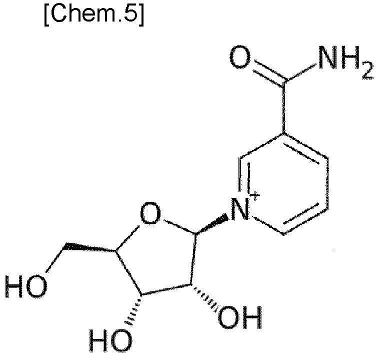 ou le dihydronicotinamide riboside (noté -NR-H) de formule :

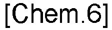

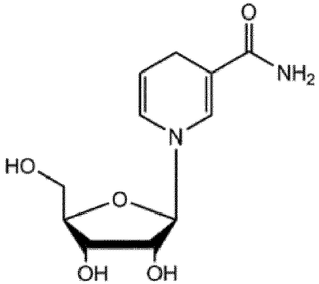

Avantageusement, le nicotinamide mononucléotide (NMN), un de ses dérivés pharmaceutiquement acceptables ou un de ses sels pharmaceutiquement acceptables, peut être administré par voie orale, oculaire, sublinguale, parentérale, transcutanée, vaginale, péridurale, intravésicale, rectale ou inhalation.

De préférence, le NMN, un de ses dérivés pharmaceutiquement acceptables ou un de ses sels pharmaceutiquement acceptables, est administré par voie orale ou parentérale.

Le NMN, un de ses dérivés pharmaceutiquement acceptables ou un de ses sels pharmaceutiquement acceptables, est utilisé dans le traitement et/ou la prévention de la douleur nociceptive viscérale chez des mammifères, de préférence des humains.

La douleur n'est pas une douleur neuropathique.

Avantageusement, le NMN, un de ses dérivés pharmaceutiquement acceptables ou un de ses sels pharmaceutiquement acceptables, est utilisé pour réduire l'allodynie.

Avantageusement, le NMN, un de ses dérivés pharmaceutiquement acceptables ou un de ses sels pharmaceutiquement acceptables, est utilisé pour réduire l'hyperalgésie.

La douleur est une douleur viscérale.

Avantageusement, la douleur est une douleur de l'appareil urogénital.

Avantageusement, la douleur est une douleur causée par une infection urinaire.

Avantageusement, le NMN, un de ses dérivés pharmaceutiquement acceptables ou un de ses sels pharmaceutiquement acceptables, est utilisé en combinaison avec au moins un autre agent thérapeutique.

Avantageusement, l'au moins un agent thérapeutique supplémentaire est choisi parmi les antibiotiques, les antifongiques, les antiviraux et leurs combinaisons.

Avantageusement, l'au moins un agent thérapeutique est un antalgique.

Avantageusement, l'antalgique est choisi parmi le paracétamol, l'aspirine, les anti-inflammatoires non stéroïdiens, les dérivés de la cortisone et leurs combinaisons.

Avantageusement, l'anti-inflammatoire non stéroïdien est choisi parmi l'ibuprofène, le kétoprofène, le naproxène, l'alminoprofène l'acéclofénac, l'acide méfénamique, l'acide niflumique, l'acide tiaprofénique, le célécoxib, le dexkétoprofène, le diclofénac, l'étodolac, l'étoricoxib, le fénoprofène, le flurbiprofène, l'indométacine, le méloxicam, le nabumétone, le piroxicam, le sulindac, le ténoxicam et leurs combinaisons.

Avantageusement, le dérivé de la cortisone est choisi parmi la bétaméthasone, la ciprofloxacine, le cortivazol, la dexaméthasone, la fludrocortisone, le methylprednisolone, le prednisolone et la triamcinolone et leurs combinaisons.

Avantageusement, l'antalgique est choisi parmi la codéine, la dihydrocodéine, le tramadol et leurs combinaisons.

Avantageusement, l'antalgique est choisi parmi la morphine, la buprénorphine, le fentanyl, l'hydromorphone, la nalbuphine, l'oxycodone, la péthidine et leurs combinaisons.

La présente invention porte également sur une composition comprenant du nicotinamide mononucléotide, un de ses dérivés pharmaceutiquement acceptables ou un de ses sels pharmaceutiquement acceptables et au moins un excipient pharmaceutiquement acceptable pour son utilisation dans la prévention et/ou le traitement de la douleur tel qu'exposé ci-dessus.

Avantageusement, la composition selon l'invention se présente sous la forme d'un comprimé, d'une gélule, d'un sachet, d'un granulé, d'une capsule molle, d'une pastille, d'un lyophilisat, d'une suspension, d'un gel, d'un sirop, d'une solution, d'une émulsion eau/huile, d'une émulsion huile/eau, d'une huile, d'une crème, d'un lait, d'une pulvérisation, d'une pommade, d'une ampoule, d'un suppositoire, d'un collyre, d'une ovule vaginale, d'une capsule vaginale, d'un liquide pour inhalation, d'un inhalateur à poudre sèche, d'un inhalateur pressurisé à valve doseuse.

De préférence, la composition selon l'invention se présente sous la forme d'une gélule gastro-résistante ou d'un comprimé sublingual.

Avantageusement, la composition selon l'invention est une composition pharmaceutique.

Avantageusement, la composition selon l'invention est un complément alimentaire.

La présente invention porte également sur une composition comprenant du nicotinamide mononucléotide, un de ses dérivés pharmaceutiquement acceptables ou un de ses sels pharmaceutiquement acceptables, au moins un excipient pharmaceutiquement acceptable et au moins un agent thérapeutique supplémentaire pour son utilisation dans la prévention et/ou le traitement de la douleur tel qu'exposé ci-dessus.

### DÉFINITIONS

Dans la présente invention, les termes suivants ont la signification suivante.

Sauf indication contraire, la nomenclature des substituants qui ne sont pas explicitement définis dans la présente invention est obtenue en nommant la partie terminale de la fonctionnalité suivie de la fonctionnalité adjacente vers le point d'attache.

« Alkyle » par lui-même ou en tant que partie d'un autre substituant, désigne un radical hydrocarbyle de formule CnH2n+1 dans laquelle n'est un nombre supérieur ou égal à 1. En général, les groupes alkyles de cette invention comprennent de 1 à 12 atomes de carbone, de préférence de 1 à 8 atomes de carbone, plus préférablement de 1 à 6 atomes de carbone, encore plus préférablement de 1 à 2 atomes de carbone. Les groupes alkyles peuvent être linéaires ou ramifiés et peuvent être substitués comme indiqué dans la présente invention. Les alkyles convenant à la mise en oeuvre de l'invention peuvent être choisis parmi methyle, ethyle, n-propyle, i-propyle, n- butyle, i-butyle, s-butyle and t-butyle, pentyle et ses isomères tels que n-pentyle et iso-pentyle, et hexyl eet ses isomères tels que n-hexyle et iso-hexyle, heptyle et ses isomères (par exemple n-heptyle, iso-heptyle), octyle et ses isomères (par exemple n-octyle, iso-octyle), nonyle et ses isomères (par exemple n-nonyle, iso-nonyle), décyle et ses isomères (par exemple n-décyle, iso-décyle), undécyle et ses isomères, dodécyle et ses isomères. De préférence, les groupes alkyles peuvent être choisis parmi methyle, ethyle, n-propyle, i-propyle, n-butyle, i-butyle, s-butyle, t-butyle, n-pentyle, n-hexyle, n-heptyle, n-octyle, n-nonyle et n-decyle. Les groupes alkyles saturés et ramifiés peuvent être choisis, de manière non limitative, parmi isopropyle, sec-butyle, isobutyle, tert-butyle, isopentyle, 2-methylbutyle, 3-methylbutyle, 2-methylpentyle, 3-methylpentyle, 4-methylpentyle, 2-methylhexyle, 3-methylhexyle, 4-methylhexyle, 5-methylhexyle, 2,3-dimethylbutyle, 2,3-dimethylpentyle, 2,4-dimethylpentyle, 2,3-dimethylhexyle, 2,4-dimethylhexyle, 2,5-dimethylhexyle, 2,2-dimethylpentyle, 2,2-dimethylhexyle, 3,3-dimtheylpentyle, 3,3-dimethylhexyle, 4,4-dimethylhexyle, 2-ethylpentyle, 3-ethylpentyle, 2-ethylhexyle, 3-ethylhexyle, 4-ethylhexyle, 2-methyl-2-ethylpentyle, 2-methyl-3-ethylpentyle, 2-methyl-4-ethylpentyle, 2-methyl-2-ethylhexyle, 2-methyl-3-ethylhexyle, 2-methyl-4-ethylhexyle, 2,2-diethylpentyle, 3,3-diethylhexyle, 2,2-diethylhexyle, et 3,3-diethylhexyle. Les groupes alkyle préférés sont les suivants : méthyle, éthyle, n-propyle, i-propyle, n-butyle, i-butyle, s-butyle et t-butyle. Les Cx-Cy-alkyles désignent les groupes alkyles qui comprennent de x à y atomes de carbone.

Lorsque le suffixe "ene" ("alkylène") est utilisé en conjonction avec un groupe alkyle, cela signifie que le groupe alkyle tel que défini ici a deux liaisons simples comme points d'attache à d'autres groupes. Le terme "alkylène" comprend le méthylène, l'éthylène, le méthylméthylène, le propylène, l'éthyléthylène et le 1,2-diméthyléthylène.

Le terme "alcényle" tel qu'il est utilisé ici se réfère à un groupe hydrocarbyle insaturé, qui peut être linéaire ou ramifié, comprenant une ou plusieurs doubles liaisons carbone-carbone. Les groupes alcényles appropriés comprennent entre 2 et 12 atomes de carbone, de préférence entre 2 et 8 atomes de carbone, et encore plus préférablement entre 2 et 6 atomes de carbone. Des exemples de groupes alcényles sont l'éthényle, le 2-propényle, le 2-butényle, le 3-butényle, le 2-pentényle et ses isomères, le 2-hexényle et ses isomères, le 2,4-pentadiényle et les groupes similaires.

0067] Le terme "alcynyle", tel qu'il est utilisé ici, désigne une classe de groupes hydrocarbyle insaturés monovalents, dans lesquels l'insaturation résulte de la présence d'une ou plusieurs triple(s) liaison(s) carbone-carbone. Les groupes alcynyle ont généralement, et de préférence, le même nombre d'atomes de carbone que celui décrit ci-dessus pour les groupes alcényle. Des exemples non limitatifs de groupes alcynyle sont l'éthynyle, le 2-propynyle, le 2-butynyle, le 3-butynyle, le 2-pentynyle et ses isomères, le 2-hexynyle et ses isomères, etc.

« Alcoxy » désigne un groupe alkyle tel que défini ci-dessus, qui est attaché à une autre partie par un atome d'oxygène. Les exemples de groupes alcoxy comprennent les groupes méthoxy, isopropoxy, éthoxy, tert-butoxy, et autres. Les groupes alcoxy peuvent être éventuellement substitués par un ou plusieurs substituants. Les groupes alcoxy inclus dans les composés de cette invention peuvent être éventuellement substitués par un groupe solubilisant.

« Aryle », tel qu'il est utilisé ici, désigne un groupe hydrocarbyle aromatique polyinsaturé ayant un seul cycle (par exemple phényle) ou plusieurs cycles aromatiques fusionnés ensemble (par exemple naphtyle) ou liés par covalence, contenant généralement 5 à 18 atomes, de préférence 5 à 12, de manière davantage préférée de 6 à 10, dont au moins un cycle est aromatique. Le cycle aromatique peut éventuellement comprendre un ou deux cycles supplémentaires (cycloalkyle, hétérocyclyle ou hétéroaryle) qui y sont fusionnés. L'aryle est également destiné à inclure les dérivés partiellement hydrogénés des systèmes carbocycliques énumérés ici. Les exemples d'aryle comprennent le phényle, le biphénylyle, le biphénylényle, le 5- ou 6-tétralinyle, le naphtalène-1- ou -2-yle, le 4-, 5-, 6 ou 7-indényle, le 1- 2-, 3-, 4- ou 5-acénaphtylényle, le 3-, 4- ou 5-acénaphtényle, 1- ou 2-pentalényle, 4- ou 5-indanyle, 5-, 6-, 7- ou 8-tétrahydronaphtyle, 1,2,3,4-tétrahydronaphtyle, 1,4-dihydronaphtyle, 1-, 2-, 3-, 4- ou 5-pyrényle.

Lorsqu'au moins un atome de carbone dans un groupe aryle est remplacé par un hétéroatome, le cycle résultant est appelé ici cycle « hétéroaryle ».

« Alkylaryle » désigne un groupe aryle substitué par un groupe alkyle.

« Acide aminé » désigne un acide carboxylique alpha-aminé, c'est-à-dire une molécule comprenant un groupe fonctionnel acide carboxylique et un groupe fonctionnel amine en position alpha du groupe acide carboxylique, par exemple un acide aminé protéinogène ou un acide aminé non protéinogène.

« Acide aminé protéinogène » désigne un acide aminé qui est incorporé dans les protéines lors de la traduction de l'ARN messager par les ribosomes dans les organismes vivants, c'est-à-dire Alanine (ALA), Arginine (ARG), Asparagine (ASN), Aspartate (ASP), Cystéine (CYS), Glutamate (acide glutamique) (GLU), Glutamine (GLN), Glycine (GLY), Histidine (HIS), Isoleucine (ILE), Leucine (LEU), Lysine (LYS), Méthionine (MET), Phénylalanine (PHE), Proline (PRO), Pyrrolysine (PYL), Sélénocystéine (SEL), Sérine (SER), Thréonine (THR), Tryptophane (TRP), Tyrosine (TYR) ou Valine (VAL).

« Acide aminé non protéinogène » tel qu'il est utilisé ici fait référence à un acide aminé qui n'est pas naturellement codé ou trouvé dans le code génétique d'un organisme vivant. Des exemples non limitatifs d'acide aminé non protéinogène sont l'ornithine, la citrulline, l'argininosuccinate, l'homosérine, l'homocystéine, l'acide cystéine-sulfinique, l'acide 2-aminomuconique, δ-aminolevulinic acid, β-alanine, cystathionine, γ-aminobutyrate, DOPA, 5-hydroxytryptophane, D-sérine, acide iboténique, α-aminobutyrate, 2-aminoisobutyrate, D-leucine, D-valine, D-alanine ou D-glutamate

Le terme "cycloalkyle" tel qu'il est utilisé ici est un groupe alkyle cyclique, c'est-à-dire un groupe hydrocarbyle monovalent, saturé ou insaturé, ayant 1 ou 2 structures cycliques. Le terme "cycloalkyle" inclut les groupes hydrocarbyle monocycliques ou bicycliques. Les groupes cycloalkyle peuvent comprendre 3 atomes de carbone ou plus dans le cycle et généralement, selon la présente invention, comprendre de 3 à 10, plus préférablement de 3 à 8 atomes de carbone et encore plus préférablement de 3 à 6 atomes de carbone. Les exemples de groupes cycloalkyle comprennent, sans s'y limiter, le cyclopropyle, le cyclobutyle, le cyclopentyle, le cyclohexyle, le cyclopropyle étant particulièrement préféré.

Par « excipient pharmaceutiquement acceptable », il est fait référence à un véhicule ou un support inerte utilisé en tant que solvant ou diluant dans lequel le principe actif est formulé et/ou administré, et qui ne produit pas une réaction indésirable, allergique ou autre lorsqu'il est administré à un animal, de préférence un être humain. Cela comprend tous les solvants, milieux de dispersion, revêtements, agents antibactériens et antifongiques, agents isotoniques, retardants d'absorption et autres ingrédients similaires. Pour l'administration humaine, les préparations doivent répondre à des normes de stérilité, de sécurité générale et de pureté, telles que requises par les offices de régulation, tels que par exemple la FDA ou l'EMA. Au sens de l'invention, « excipient pharmaceutiquement acceptable » inclut tous les excipients pharmaceutiquement acceptables ainsi que tous les supports, diluants, et/ou adjuvants pharmaceutiquement acceptables.

« Halogène » ou « halo » signifie fluoro, chloro, bromo ou iodo. Les groupes halo préférés sont le fluoro et le chloro.

« Haloalkyle » seul ou en combinaison, désigne un radical alkyle ayant la signification telle que définie ci-dessus, dans lequel un ou plusieurs atomes d'hydrogène sont remplacés par un halogène tel que défini ci-dessus. Parmi les exemples de tels radicaux halogénoalkyle, on peut citer le chlorométhyle, le 1-bromoéthyle, le fluorométhyle, le difluorométhyle, le trifluorométhyle, le 1,1,1-trifluoroéthyle et les radicaux similaires. Cx-Cy-haloalkyle et Cx-Cy-alkyle désignent des groupes alkyles qui comprennent de x à y atomes de carbone. Les groupes halogénoalkyle préférés sont le difluorométhyle et le trifluorométhyle.

« Hétéroalkyle » désigne un groupe alkyle tel que défini ci-dessus, dans lequel un ou plusieurs atomes de carbone sont remplacés par un hétéroatome choisi parmi les atomes d'oxygène, d'azote et de soufre. Dans les groupes hétéroalkyle, les hétéroatomes sont liés le long de la chaîne alkyle uniquement à des atomes de carbone, c'est-à-dire que chaque hétéroatome est séparé de tout autre hétéroatome par au moins un atome de carbone. Toutefois, les hétéroatomes d'azote et de soufre peuvent éventuellement être oxydés et les hétéroatomes d'azote peuvent éventuellement être quaternisés. Un hétéroalkyle est lié à un autre groupe ou à une autre molécule uniquement par un atome de carbone, c'est-à-dire que l'atome de liaison n'est pas choisi parmi les hétéroatomes inclus dans le groupe hétéroalkyle.

Le terme "hétéroaryle" tel qu'il est utilisé ici, seul ou en tant que partie d'un autre groupe, désigne, sans s'y limiter, des cycles aromatiques de 5 à 12 atomes de carbone ou des systèmes cycliques contenant 1 ou 2 cycles qui sont fusionnés ou liés par covalence, contenant généralement 5 ou 6 atomes ; dont au moins un est aromatique, dans lequel un ou plusieurs atomes de carbone dans un ou plusieurs de ces cycles sont remplacés par des atomes d'oxygène, d'azote et/ou de soufre, les hétéroatomes d'azote et de soufre pouvant éventuellement être oxydés et les hétéroatomes d'azote pouvant éventuellement être quaternisés. Ces cycles peuvent être fusionnés à un cycle aryle, cycloalkyle, hétéroaryle ou hétérocyclyle. Parmi les exemples non limitatifs de tels hétéroaryles, on peut citer furanyle, thiophényle, pyrazolyle, imidazolyle, oxazolyle, isoxazolyle, thiazolyle, isothiazolyle, triazolyle, oxadiazolyle, thiadiazolyle, tétrazolyle, oxatriazolyle, thiatriazolyle, pyridinyle, pyrimidyle, pyrazinyle, pyridazinyle, oxazinyle, dioxinyle, thiazinyle, triazinyle, imidazo [2, 1 -b] [1 ,3] thiazolyle, thiéno [3 ,2-b] furanyle, thiéno [3 ,2-b] thiophényle, thiéno [2,3-d] [I,3] thiazolyle, thiéno [2,3-d] imidazolyle, tétrazolo [I,5-a] pyridinyle, indolyle, indolizinyle, isoindolyle, benzofuranyle, isobenzofuranyle, benzothiophényle, isobenzothiophényle, indazolyle, benzimidazolyle, 1,3-benzoxazolyle, 1,2- benzisoxazolyle, 2,1-benzisoxazolyle, 1,3-benzothiazolyle, 1,2-benzoisothiazolyle, 2,1-benzoisothiazolyle, benzotriazolyle, 1,2,3-benzoxadiazolyle, 2,1,3- benzoxadiazolyle, 1,2,3-benzothiadiazolyle, 2,1,3-benzothiadiazolyle, thiénopyridinyle, purinyle, imidazo[I,2-a]pyridinyl, 6-oxo-pyridazin-I(6H)-yl, 2- oxopyridin-I(2H)-yl, 6-oxo-pyridazin-I(6H)-yl, 2-oxopyridin-I(2H)-yl, 1,3-benzodioxolyl, quinolinyl, isoquinolinyl, cinnolinyl, quinazolinyl, quinoxalinyl.

Lorsqu'au moins un atome de carbone dans un groupe cycloalkyle est remplacé par un hétéroatome, le cycle résultant est appelé ici "hétérocycloalkyle" ou "hétérocyclyle".

Les termes "hétérocyclyle", "hétérocycloalkyle" ou "hétérocyclo", tels qu'ils sont utilisés ici par eux-mêmes ou en tant que partie d'un autre groupe, désignent des groupes cycliques non aromatiques, totalement saturés ou partiellement insaturés (par exemple, monocyclique de 3 à 7 chaînons, bicyclique de 7 à 11 chaînons ou contenant un total de 3 à 10 atomes de cycle) qui ont au moins un hétéroatome dans au moins un cycle contenant un atome de carbone. Chaque cycle du groupe hétérocyclique contenant un hétéroatome peut avoir 1, 2, 3 ou 4 hétéroatomes choisis parmi les atomes d'azote, d'oxygène et/ou de soufre, où les hétéroatomes d'azote et de soufre peuvent éventuellement être oxydés et les hétéroatomes d'azote peuvent éventuellement être quaternisés. N'importe lequel des atomes de carbone du groupe hétérocyclique peut être substitué par un oxo (par exemple pipéridone, pyrrolidinone). Le groupe hétérocyclique peut être attaché à n'importe quel hétéroatome ou atome de carbone du cycle ou du système cyclique, lorsque la valence le permet. Les cycles des hétérocycles à plusieurs cycles peuvent être fusionnés, pontés et/ou reliés par un ou plusieurs atomes spiro. Les groupes hétérocycliques exemplaires non limitatifs comprennent les groupes oxétanyle, pipéridinyle, azétidinyle, 2-imidazolinyle, pyrazolidinyle, imidazolidinyle, isoxazolinyle, oxazolidinyle, isoxazolidinyle, thiazolidinyle, isothiazolidinyle, pipéridinyle, 3H- indolyle, indolinyle, isoindolinyle, 2-oxopipérazinyle, pipérazinyle, homopipérazinyle, 2-pyrazolinyle, 3-pyrazolinyle, tétrahydro-2H-pyranyle, 2H-pyranyle, 4H-pyranyle, 3,4-dihydro-2H-pyranyle, 3-dioxolanyle, 1,4-dioxanyle, 2, 5-dioximidazolidinyle, 2-oxopipéridinyle, 2-oxopyrrolodinyle, indolinyle, tétrahydropyranyle, tétrahydrofuranyle, tétrahydroquinolinyle, tétrahydroisoquinolin-1-yle, tétrahydroisoquinolin-2-yle, tétrahydroisoquinolin-3-yle, tétrahydroisoquinoléine-4-yl, thiomorpholine-4-yl, oxyde de thiomorpholine-4-ylsulf, thiomorpholine-4-ylsulfone, 1,3- dioxolanyl, 1,4-oxathianyl, 1H-pyrrolizinyl, tétrahydro-I,I-dioxothiophényl, N- formylpipérazinyl et morpholine-4-yl.

Le terme "précurseur" tel qu'il est utilisé ici désigne également les dérivés pharmacologiquement acceptables des composés de formule (I) ou (II) tels que les esters dont le produit de biotransformation in vivo est le médicament actif. Les précurseurs sont caractérisés par une biodisponibilité accrue et sont facilement métabolisés en composés actifs in vivo. Les précurseurs appropriés aux fins de l'invention comprennent notamment les esters carboxyliques, en particulier les esters alkyliques, les esters aryliques, les esters acyloxyalkyliques et les esters carboxyliques de dioxolène ; les esters d'acide ascorbique.

« Pharmaceutiquement acceptable » signifie approuvé ou susceptible d'être approuvé par un organisme de réglementation ou inscrit dans une pharmacopée reconnue pour l'utilisation chez les animaux, et plus préférablement chez l'homme. Il peut s'agir d'une substance qui n'est pas indésirable sur le plan biologique ou autre, c'est-à-dire que la substance peut être administrée à un individu sans provoquer d'effets biologiques indésirables ou d'interactions délétères avec l'un des composants de la composition dans laquelle elle est contenue. De préférence, un sel ou un excipient « pharmaceutiquement acceptable » désigne tout sel ou tout excipient autorisé par la Pharmacopée européenne (notée « Ph. Eur. ») et la pharmacopée américaine (désignée par « United States Pharmacopeia (USP) » en anglais).

Le terme "principe actif' désigne une molécule ou une substance dont l'administration à un sujet ralentit ou arrête la progression, l'aggravation ou la détérioration d'un ou de plusieurs symptômes d'une maladie ou d'un état ; soulage les symptômes d'une maladie ou d'un état ; guérit une maladie ou un état. Selon l'un de ces modes de réalisation, l'ingrédient thérapeutique est une petite molécule, naturelle ou synthétique. Selon une autre, l'ingrédient thérapeutique est une molécule biologique comme par exemple un oligonucléotide, un siRNA, un miRNA, un fragment d'ADN, un aptamère, un anticorps et autres. Les « sels pharmaceutiquement acceptables » comprennent les sels d'addition d'acides et de bases de ces sels. Les sels d'addition d'acide appropriés sont formés à partir d'acides qui forment des sels non toxiques. Il s'agit par exemple de l'acétate, l'adipate, l'aspartate, le benzoate, le bésylate, le bicarbonate/carbonate, le bisulfate/sulfate, le borate, le camsylate, le citrate, le cyclamate, l'édisylate, l'esylate, le formiate, le fumarate, le gluceptate, le gluconate, le glucuronate, l'hexafluorophosphate, l'hibenzate, le chlorhydrate/chlorure, le bromhydrate/bromure, l'hydroiodure/iodure, iséthionate, lactate, malate, maléate, malonate, mésylate, méthylsulfate, naphtylate, 2-napsylate, nicotinate, nitrate, orotate, oxalate, palmitate, pamoate, phosphate/hydrogénophosphate/dihydrogénophosphate, pyroglutamate, saccharate, stéarate, succinate, tannate, tartrate, tosylate, trifluoroacétate et sels de xinofoate. Les sels basiques appropriés sont formés à partir de bases qui forment des sels non toxiques. On peut citer comme exemples les sels d'aluminium, d'arginine, de benzathine, de calcium, de choline, de diéthylamine, de diolamine, de glycine, de lysine, de magnésium, de méglumine, d'olamine, de potassium, de sodium, de trométhamine, de 2-(diéthylamino)éthanol, d'éthanolamine, de morpholine, de 4-(2-hydroxyéthyl)morpholine et de zinc. Des hémisels d'acides et de bases peuvent également être formés, par exemple, des hémisulfates et des sels de calcium chimique. Les sels pharmaceutiquement acceptables préférés sont le chlorhydrate/chlorure, le bromure/hydrobromure, le bisulfate/sulfate, le nitrate, le citrate et l'acétate.

Les sels pharmaceutiquement acceptables peuvent être préparés par une ou plusieurs de ces méthodes :
i. en faisant réagir le composé avec l'acide souhaité ;
ii. en faisant réagir le composé avec la base souhaitée ;
iii. en éliminant un groupe protecteur labile en milieu acide ou basique d'un précurseur approprié du composé ou en ouvrant le cycle d'un précurseur cyclique approprié, par exemple une lactone ou un lactame, en utilisant l'acide désiré ; ou
iv. en transformant un sel du composé en un autre par réaction avec un acide approprié ou au moyen d'une colonne d'échange d'ions appropriée.

Toutes ces réactions sont généralement effectuées en solution. Le sel peut précipiter de la solution et être recueilli par filtration ou peut être récupéré par évaporation du solvant. Le degré d'ionisation du sel peut varier de complètement ionisé à presque non ionisé.

« Solvate » est utilisé ici pour décrire un complexe moléculaire comprenant le composé de l'invention et une ou plusieurs molécules de solvant pharmaceutiquement acceptables, par exemple, l'éthanol.

Le terme "substituant" ou "substitué" signifie qu'un radical hydrogène sur un composé ou un groupe est remplacé par tout groupe souhaité qui est substantiellement stable dans les conditions de la réaction sous une forme non protégée ou lorsqu'il est protégé par un groupe protecteur. Les exemples de substituants préférés comprennent, sans s'y limiter, un halogène (chloro, iodo, bromo ou fluoro) ; un alkyle ; un alcényle ; un alcynyle, tel que décrit ci-dessus ; un hydroxy ; un alcoxy ; un nitro ; un thiol ; un thioéther ; une imine ; un cyano ; un amido ; un phosphonato ; une phosphine ; un carboxyle ; un thiocarbonyle ; un sulfonyle ; un sulfonamide ; une cétone ; un aldéhyde ; un ester ; un oxygène (-O) ; un haloalkyle (par ex, trifluorométhyle) ; un cycloalkyle, qui peut être monocyclique ou polycyclique condensé ou non condensé (par exemple, cyclopropyle, cyclobutyle, cyclopentyle ou cyclohexyle), ou un hétérocycloalkyle, qui peut être monocyclique ou polycyclique condensé ou non condensé (par exemple, pyrrolidinyle, pipéridinyle, pipérazinyle, morpholinyle ou thiazinyle), monocyclique ou polycyclique fusionné ou non fusionné, aryle ou hétéroaryle (par exemple, aryle, hétéroaryle, pyrrolidinyle, pipéridinyle, pipérazinyle, morpholinyle ou thiazinyle), monocyclique ou polycyclique fusionné ou non fusionné (par exemple, aryle, hétéroaryle, pyrrolidinyle, pipéridinyle, pipérazinyle, morpholinyle ou thiazinyle), phényle, naphtyle, pyrrolyle, indolyle, furanyle, thiophényle, imidazolyle, oxazolyle, isoxazolyle, thiazolyle, triazolyle, tétrazolyle, pyrazolyle, pyridyle, quinolinyle, isoquinolinyle, acridinyle, pyrazinyle, pyridazinyle, pyrimidinyle, benzimidazolyle, benzothiophényle ou benzofuranyle) ; amino (primaire, secondaire ou tertiaire) ; CO₂CH₃ ; CONH2 ; OCH₂CONH₂ ; NH₂ ; SO₂NH₂ ; OCHF₂ ; CF₃ ; OCF₃ ; et ces groupements peuvent également être éventuellement substitués par une structure ou un pont annulaire fusionné, par exemple -OCH₂O-. Ces substituants peuvent éventuellement être encore substitués par un substituant choisi parmi ces groupes. Dans certaines représentations, le terme "substituant" ou l'adjectif "substitué" désigne un substituant choisi dans le groupe constitué par un alkyle, un alcényle, un alcynyle, un cycloalkyle, un cycloalcényle, un hétérocycloalkyle, un aryle, un hétéroaryle, un arylalkyle, un hétéroarylalkyle, un haloalkyle, -C(O)NR₁₁R₁₂, - NR₁₃C(O)R₁₄, un halo, -OR₁₃, cyano, nitro, un haloalcoxy, -C(O)R₁₃, -NR₁₁R₁₂, - SR₁₃, -C(O)OR'₁₃, -OC(O)R₁₃, -NR₁₃C(O)NR₁₁R₁₂, -OC(O)NR₁₁R₁₂, - NR₁₃C(O)OR₁₄, -S(O)rR13, -NR₁₃S(O)rR₁₄, -OS(O)rR₁₄, S(O)rNR₁₁R₁₂, -O, -S, et - N-R₁₃, où r est 1 ou 2 ; R₁₁ et R₁₂, pour chaque occurrence, sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocycloalkyle éventuellement substitué, un aryle éventuellement substitué, un hétéroaryle éventuellement substitué, un arylalkyle éventuellement substitué, ou un hétéroarylalkyle éventuellement substitué ; ou R₁₁ et R₁₂ pris ensemble avec l'azote auquel ils sont attachés sont un hétérocycloalkyle éventuellement substitué ou un hétéroaryle éventuellement substitué ; et R₁₃ et R₁₄ pour chaque occurrence sont, indépendamment, H, un alkyle éventuellement substitué, un alcényle éventuellement substitué, un alcynyle éventuellement substitué, un cycloalkyle éventuellement substitué, un cycloalcényle éventuellement substitué, un hétérocycloalkyle éventuellement substitué, un aryle éventuellement substitué, un hétéroaryle éventuellement substitué, un arylalkyle éventuellement substitué, ou un hétéroarylalkyle éventuellement substitué. Dans certaines variantes, le terme "substituant" ou l'adjectif "substitué" désigne un groupe solubilisant.

Le terme « administration », ou une variante de ce terme (par exemple, « administrer »), signifie fournir le principe actif, seul ou dans le cadre d'une composition pharmaceutiquement acceptable, au patient chez qui/à qui l'état, le symptôme ou la maladie doit être traité ou prévenu.

« Traiter », « soigner » et « traitement », tels qu'ils sont utilisés dans la présente invention, sont censés inclure le soulagement, l'atténuation ou l'ablation d'un état ou d'une maladie et/ou de ses symptômes associés.

« Prévenir », « empêcher » et « prévention », tels qu'ils sont utilisés dans la présente invention, font référence à une méthode permettant de retarder ou d'empêcher l'apparition d'un état ou d'une maladie et/ou de ses symptômes connexes, d'empêcher un patient de contracter un état ou une maladie, ou de réduire le risque qu'un patient contracte un état ou une maladie.

Les liaisons d'un carbone asymétrique peuvent être représentées ici en utilisant un triangle plein ( 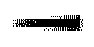 ) un triangle pointillé ( 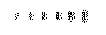 ) ou une ligne en zigzag ( 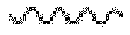 ).

### DESCRIPTION DETAILLEE DE L'INVENTION

La présente invention a pour objet le nicotinamide mononucléotide (NMN), un de ses dérivés pharmaceutiquement acceptables ou un de ses sels pharmaceutiquement acceptables, tel que défini dans la revendication 1, pour son utilisation dans la prévention et/ou le traitement de la douleur nociceptive viscérale qui n'est pas une douleur neuropathique.

La présente invention a également pour objet une composition comprenant le NMN, un de ses dérivés pharmaceutiquement acceptables ou un de ses sels pharmaceutiquement acceptables tel que défini dans la revendication 1, et au moins un excipient pharmaceutiquement acceptable pour son utilisation dans la prévention et/ou le traitement de la douleur nociceptive viscérale qui n'est pas une douleur neuropathique.

Le nicotinamide adénine dinucléotide (NAD) est une coenzyme présente dans toutes les cellules vivantes. Le NAD existe dans la cellule soit sous sa forme oxydée NAD+, soit sous sa forme réduite NADH. Le rôle de NAD est celui d'un transporteur d'électrons intervenant dans les réactions d'oxydoréduction du métabolisme. Le NAD intervient en outre dans de nombreux processus cellulaires tels que la ribosylation de l'ADP dans le cadre de modifications post-traductionnelles des protéines.

Le NAD peut être synthétisé de novo par la cellule à partir d'acides aminés comme le tryptophane ou l'aspartate. Cependant, cette synthèse est marginale car la principale voie de synthèse de NAD est la voie de sauvetage par laquelle la cellule, et principalement le noyau cellulaire, recycle des composés pour reformer le NAD à partir de précurseurs. Les précurseurs du NAD comprennent la niacine, le nicotinamide riboside, le nicotinamide mononucléotide et le nicotinamide.

Le NMN (I-J) est un des composés permettant la synthèse de NAD par la voie de sauvetage et a pour formule :

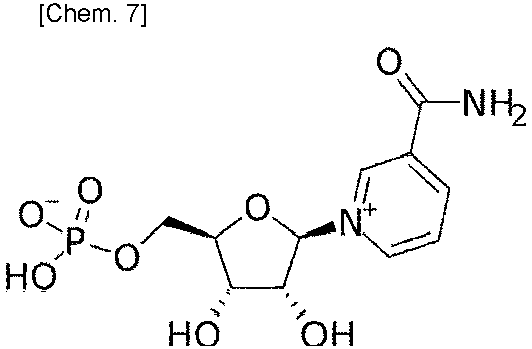

Les inventeurs ont en effet démontré que l'utilisation du NMN, un de ses dérivés pharmaceutiquement acceptables ou un de ses sels pharmaceutiquement acceptables, et de la composition selon l'invention permettait d'obtenir un effet sur la douleur comparable aux médicaments couramment utilisés pour traiter la douleur. Plus exactement, l'utilisation du NMN et de la composition selon l'invention conformément à l'invention permet de prévenir la douleur car elle permet de réduire l'intensité de la douleur développée en réponse à un stimulus douloureux. L'utilisation de NMN, un de ses dérivés pharmaceutiquement acceptables ou un de ses sels pharmaceutiquement acceptables, et de la composition selon l'invention est également efficace pour traiter une douleur déjà présente.

De plus, l'utilisation de NMN, molécule naturellement présente dans le corps, présente de nombreux avantages. Notamment, le NMN et la composition selon l'invention sont bien tolérés par les patients. L'utilisation de NMN et de la composition selon l'invention n'induit en effet aucune allergie chez les patients. De plus, l'utilisation de NMN et de la composition selon l'invention ne provoque pas les effets secondaires fréquemment rencontrés avec les antalgiques conventionnels tels que, par exemple, les ulcères, la toxicité hépatique, la fluidification du sang, la somnolence, les nausées et vomissements.

Le NMN n'induit en outre aucun phénomène de dépendance physique ou psychologique, contrairement aux antalgiques comprenant de la morphine ou les dérivés de l'opium. L'utilisation de NMN et de la composition selon l'invention pour prévenir et/ou traiter la douleur est donc sûre pour les patients.

Le NMN et la composition selon l'invention peuvent donc être utilisés chez l'enfant et l'adulte. Le NMN est en effet bien toléré par les enfants. Dans le contexte de l'invention, on considère qu'un patient est un enfant lorsque son âge est inférieur à 16 ans et qu'il est un adulte à partir de 16 ans.

Dans un mode de réalisation préféré, le NMN se trouve sous la forme d'un zwitterion. On entend par « zwitterion » une espèce chimique moléculaire possédant des charges électriques de signe opposé et situées, en général, sur des atomes non adjacents de la molécule.

L'excipient pharmaceutiquement acceptable peut être choisi parmi un agent de charge, un lubrifiant, un arôme, un colorant, un émulsifiant, un agent de compression, un diluant, un conservateur, un gélifiant, un plastifiant, un tensioactif ou leurs combinaisons. L'homme de l'art sait quel excipient choisir en fonction de la forme galénique qu'il aura choisie.

Dans le contexte de la présente invention, un « excipient » désigne toute substance autre que le NMN dans la composition et n'ayant pas d'effet thérapeutique. L'excipient n'interagit pas sur le plan chimique avec le NMN ou tout autre agent thérapeutique supplémentaire.

Le NMN, un de ses dérivés pharmaceutiquement acceptables ou un de ses sels pharmaceutiquement acceptables, et la composition selon l'invention peuvent être administrés à une quantité thérapeutiquement efficace. Dans le contexte de l'invention, une quantité thérapeutiquement efficace signifie que la composition est administrée à un patient en quantité suffisante pour obtenir l'effet thérapeutique désiré, en l'occurrence réduire la sensation de douleur.

Dans un mode de réalisation, le NMN, un de ses dérivés pharmaceutiquement acceptables ou un de ses sels pharmaceutiquement acceptables, est utilisé dans une quantité comprise entre 0,01 mg/kg/jour et 1000 mg/kg/jour, de préférence entre1 mg/kg/jour et 100 mg/kg/jour, de manière davantage préférée entre 5 mg/kg/jour et 50 mg/kg/jour, de manière encore plus préférée entre 10 mg/kg/jour et 20 mg/kg/jour. L'homme du métier peut adapter la dose de NMN à administrer en fonction de l'âge et du poids du patient, et de l'intensité de la douleur à traiter.

Un niveau de dosage approprié peut être d'environ 0,01 à 250 mg/kg par jour, d'environ 0,05 à 100 mg/kg par jour, ou d'environ 0,1 à 50 mg/kg par jour. Dans cette fourchette, la dose peut être de 0,05 à 0,5, 0,5 à 5 ou 5 à 50 mg/kg par jour. Pour l'administration orale, les compositions sont de préférence fournies sous forme de comprimés contenant de 1,0 à 1000 milligrammes du principe actif, notamment 1,0, 5,0, 10,0, 15,0, 20,0, 25,0, 50,0, 75. 0, 100.0, 150.0, 200.0, 250.0, 300.0, 400.0, 500.0, 600.0, 750.0, 800.0, 900.0 et 1000.0 milligrammes de l'ingrédient actif pour l'ajustement symptomatique de la dose au patient à traiter. Par exemple, la posologie peut être comprise entre 100mg/jour et 5000mg/jour, de préférence entre 500mg/jour et 1000mg/jour. Les composés peuvent être administrés selon un schéma de 1 à 4 fois par jour, de préférence une, deux ou trois fois par jour, de préférence trois fois par jour. La durée du traitement dépend et est déterminée par le médecin. Elle peut aller d'un jour à un an ou même plus, de préférence d'une semaine à trois mois, plus préférablement de deux semaines à six semaines. Il sera toutefois entendu que le niveau de dose spécifique et la fréquence de dosage ainsi que la durée pour un patient donné peuvent varier et dépendront de divers facteurs, notamment l'activité du composé spécifique employé, la stabilité métabolique et la durée d'action de ce composé, l'âge, le poids corporel, l'état de santé général, le sexe, le régime alimentaire, le mode et le moment d'administration, le taux d'excrétion, la combinaison de médicaments, et l'hôte soumis au traitement.

Le NMN, un de ses dérivés pharmaceutiquement acceptables ou un de ses sels pharmaceutiquement acceptables, peut être administré à une dose quotidienne de 10 mg/kg, avec un minimum de 50 mg/jour et un maximum de 1000 mg/jour.

Différentes échelles existent pour évaluer la douleur, en fonction de l'âge du patient et de son état cognitif, et sont bien connues de l'homme de l'art. Il existe des échelles pour :
- l'auto-évaluation par le patient, adulte ou enfant à partir de 4-6 ans (âge scolaire), capables de communiquer de l'intensité ou des caractéristiques de la douleur ;
- l'hétéro-évaluation, c'est-à-dire l'évaluation par les soignants, de la douleur des adultes non communiquant (personnes âgées, patients de réanimation, polyhandicapés... ) ou d'un enfant de moins de 4 ans.

Les échelles d'autoévaluation de la douleur chez l'enfant comprennent notamment l'échelle visuelle analogique verticale (EVA), l'échelle numérique (EN), la planche des visages, l'échelle des jetons et le schéma corporel sur lequel l'enfant situe sa douleur.

Les échelles d'hétéroévaluation de la douleur aigüe chez l'enfant comprennent notamment le système de codage du visage du nouveau-né (ou NFCS pour « Néonatal Facial coding sytem » en anglais), l'échelle de la douleur aigüe du nouveau-né, l'échelle basée sur l'observation du visage-jambe-activité-cri-consolabilité (ou FLACC pour «Face Legs Activity Cry Consolability » en anglais), l'échelle du confort (ou « Comfort Behavior » en anglais), l'échelle de la douleur de l'enfant prématuré (ou PIPP pour «Prématuré Infant Pain Profile » en anglais), l'échelle d'évaluation de la douleur aigüe de l'enfant en ambulatoire (ou CHEOPS pour « Children's Hospital of Eastern Ontario Pain Scale »), l'échelle Envendol. Les échelles d'hétéroévaluation de la douleur chronique chez l'enfant comprennent notamment l'échelle de la douleur et d'inconfort du nouveau-né (EDIN), l'échelle de la douleur objective (ou « Objective Pain Scale » en anglais), Amiel-Tison inversée, l'échelle de la douleur enfant Gustave-Roussy (DEGR), l'échelle de l'hétéro-évaluation de la douleur de l'enfant (HEDEN) et le questionnaire de la douleur de Saint-Antoine (QDSA).

Les échelles d'autoévaluation chez l'adulte comprennent l'échelle visuelle analogique verticale, l'échelle numérique et l'échelle verbale simple. Les échelles d'hétéroévaluation chez l'adulte comprennent le test algoplus, le test doloplus et l'évaluation comportementale chez la personne âgée (ECPA). Enfin, il existe un test spécifique pour l'évaluation de la douleur neuropathique : le test DN4.

Le NMN, un de ses dérivés pharmaceutiquement acceptables ou un de ses sels pharmaceutiquement acceptables, et la composition selon l'invention peuvent être administrés une fois par jour ou plusieurs fois par jour. Notamment, le NMN et la composition selon l'invention peuvent être administrés entre 1 et 12 fois par jour, de préférence entre 3 et 10 fois par jour, de manière davantage préférée entre 5 et 8 fois par jour.

La dose administrée et la fréquence d'administration dépendent notamment de l'intensité de la douleur ressentie par le patient.

### Utilisation

Selon la présente invention, le NMN, un de ses dérivés pharmaceutiquement acceptables ou un de ses sels pharmaceutiquement acceptables, et la composition selon l'invention sont utilisés pour prévenir et/ou traiter la douleur nociceptive viscérale. La douleur n'est pas une douleur neuropathique. La douleur est une douleur nociceptive.

Le NMN, un de ses dérivés pharmaceutiquement acceptables ou un de ses sels pharmaceutiquement acceptables, et la composition selon l'invention sont notamment utilisés pour réduire la sensibilité à la douleur. Dans un mode de réalisation, le NMN, un de ses dérivés pharmaceutiquement acceptables ou un de ses sels pharmaceutiquement acceptables, et les compositions le comprenant conformément à l'invention sont utilisés pour réduire l'allodynie. Dans le contexte de l'invention, l'allodynie est une douleur ressentie en réponse à un stimulus qui normalement ne provoque pas de douleur. En d'autres termes, en situation d'allodynie, le seuil de tolérance à la douleur du patient est réduit. L'utilisation de NMN, un de ses précurseurs pharmaceutiquement acceptables, un de ses dérivés pharmaceutiquement acceptables ou un de ses sels pharmaceutiquement acceptables, et de compositions le comprenant selon l'invention permet donc de rétablir un seuil de tolérance normal à la douleur du patient et de supprimer, ou à tout le moins de réduire, le phénomène d'allodynie. Différentes techniques bien connues de l'homme du métier sont utilisées pour évaluer l'allodynie chez l'humain. L'allodynie chez l'homme peut par exemple être mesurée par stimulation avec différents filaments (principe du test de von Frey) soit sur une plaie (surface post-opératoire), soit après injection intradermique de capsaïceine, soit après chauffage de la peau. L'allodynie peut également être mesurée en utilisant des questionnaires dans lesquels le patient indique le score de sa douleur après, par exemple, immersion de sa main dans la glace, ou après chauffage pendant un temps donné à une température donnée d'une zone ou encore après application d'une pression définie à différent endroits (bras, genou, périnée, anus...). On peut également stimuler une zone avec des stimuli connus comme étant non-douloureux et évaluer quelle est la douleur ressentie par le patient à l'aide de questionnaires.

Dans un mode de réalisation, le NMN, un de ses dérivés pharmaceutiquement acceptables ou un de ses sels pharmaceutiquement acceptables, et la composition selon l'invention sont utilisés pour réduire l'hyperalgésie. Dans le contexte de l'invention, l'hyperalgésie se définit comme une douleur exacerbée en réponse à un stimulus douloureux.

La douleur est une douleur viscérale. Dans le contexte de l'invention, la douleur viscérale résulte de l'activation des nocicepteurs des organes thoraciques, abdominaux et pelviens. De telles douleurs peuvent comprendre notamment la sensation de distension, de coupures, de brulures, et leurs combinaisons. La distension désigne tout symptôme tel que l'étirement, le spasme, la traction, la compression et la torsion. La douleur viscérale peut être causée notamment par une traction soudaine sur le mésentère, l'étirement d'une séreuse, la compression d'un viscère causant secondairement une distension, la distension d'un organe creux comme l'estomac ou l'intestin. Sur le plan clinique, la douleur viscérale peut être causée par une inflammation, une infection, une perturbation des processus mécaniques normaux tel un trouble de la mobilité gastro-intestinale, une tumeur et une ischémie.

Dans un mode de réalisation, la douleur viscérale est une douleur ressentie au niveau de l'appareil urogénital féminin ou masculin. L'appareil urogénital comprend les reins, les uretères, la vessie, l'urètre, l'appareil génital féminin, l'appareil génital masculin, les ovaires, les testicules.

Une telle douleur peut notamment être une cystite interstitielle. La cystite interstitielle ou syndrome de la vessie douloureuse est une maladie inflammatoire de la vessie qui se caractérise par des envies anormales d'uriner (envies pressantes et/ou fréquentes) et par des douleurs importantes dans le bas ventre et la vessie, au niveau de l'urètre (canal transportant l'urine de la vessie vers l'extérieur) ou au niveau du vagin chez les femmes, parfois accompagnées d'une difficulté à uriner. La sévérité des symptômes est variable d'une personne à l'autre. La douleur peut également être due à une inflammation des voies urogénitales sans origine bactérienne, fongique ou virale. Par exemple, la douleur peut être causée par une cystite non microbienne.

Dans un mode de réalisation, la douleur viscérale est une douleur causée par une infection urinaire. L'infection urinaire peut être causée par une infection bactérienne, fongique, virale ou leurs combinaisons. L'infection urinaire peut toucher plusieurs organes de l'appareil uro-génital tels que les reins, les uretères, la vessie, l'urètre et la prostate. Elle se manifeste souvent par une douleur accompagnée ou non d'une sensation de brulure lors de la miction. L'infection urinaire peut être une cystite, une urétrite, une pyélonéphrite, une prostatite ou leurs combinaisons.

La cystite est la forme d'infection urinaire la plus courante. Il s'agit d'une inflammation de la vessie. La cystite peut être provoquée par la prolifération de bactéries intestinales telles *qu'Escherichia coli,* qui sont nombreuses aux environs de l'anus. Chez la femme, les bactéries passent de la région anale à la vessie via le méat urinaire, et causent une inflammation locale. Chez les hommes, les cystites sont souvent provoquées par une infection bactérienne telle qu'une infection aux chlamydias ou aux gonocoques. La cystite peut parfois être causée par une contamination par un champignon comme *Candida albicans.*

L'urétrite désigne une inflammation de l'urètre, c'est-à-dire le conduit reliant la vessie au méat urinaire. La prostate étant située près de l'urètre masculin, l'inflammation peut également toucher la prostate, ce qui correspond à une prostatite.

La pyélonéphrite désigne l'inflammation du bassinet du rein, c'est-à-dire la cavité collectant les urines dans le rein. Elle est souvent causée par une infection bactérienne, et notamment par une cystite mal soignée. En ce cas, les bactéries remontent le long de l'uretère pour contaminer le rein.

Dans un mode de réalisation préféré, le NMN, un de ses dérivés pharmaceutiquement acceptables ou un de ses sels pharmaceutiquement acceptables, et la composition selon l'invention sont utilisées pour traiter ou prévenir la douleur causée par une cystite. Ainsi, le NMN, un de ses dérivés pharmaceutiquement acceptables ou un de ses sels pharmaceutiquement acceptables, et la composition selon l'invention permettent de traiter la douleur causée par les infections urinaires.

### Mode d'administration et forme galénique

Le NMN, un de ses dérivés pharmaceutiquement acceptables ou un de ses sels pharmaceutiquement acceptables, et la composition selon l'invention sont administrés par voie orale, oculaire, inhalation, sublinguale, intraveineuse, intraartérielle, intramusculaire, sous-cutanée, transcutanée, vaginale, péridurale, topique, intravésicale ou rectale. De préférence, le NMN et la composition selon l'invention sont administrés par voie orale.

La composition selon l'invention peut se présenter sous la forme d'un comprimé, d'une gélule, d'un sachet, d'un granulé, d'une capsule molle, d'un lyophilisat, d'une pastille, d'une suspension, d'un gel, d'un sirop, d'une solution, d'une émulsion eau/huile, d'une émulsion huile/eau, d'une huile, d'une crème, d'un lait d'une pulvérisation, d'une pommade, une ampoule, un suppositoire, un collyre, une ovule vaginale, d'une capsule vaginale, un liquide pour inhalation, d'un inhalateur à poudre sèche, d'un inhalateur pressurisé à valve doseuse. De préférence, la composition selon l'invention se présente sous la forme d'une gélule gastrorésistante ou d'un comprimé sublingual.

On entend par « gastro-résistante » une forme galénique qui ne se dissout pas dans l'estomac. De telles formes galéniques sont à libération différée c'est-à-dire qu'elles présentent un enrobage ou une composition d'enrobage résistant au pH acide de l'estomac (pH<2) pour se dissoudre dans l'intestin. Le caractère gastrorésistant est déterminé en suivant le test établi par la Pharmacopée européenne. Brièvement, le caractère gastrorésistant d'une gélule est mesuré dans de l'acide chlorhydrique 0,1 M à 37°C comme milieu de désagrégation dans un appareil à désagrégation. Ce milieu mime les conditions physicochimiques de l'estomac. Les gélules sont incubées dans ce milieu pendant 1h. La gélule ne doit présenter ni signe de désagrégation, ni fissure pouvant entraîner une perte de contenu. La gélule est ensuite incubée pendant 1h dans une solution de tampon phosphate de pH 6,8 à 37°C, cette solution mimant les conditions du milieu intestinal conformément aux recommandations de la pharmacopée européenne. La gélule doit être totalement désagrégée en moins d'une heure.

On entend par « comprimé sublingual » une forme galénique se plaçant sous la langue afin que le principe actif soit absorbé par la muqueuse sublinguale, et notamment par les veine et artère ranines.

La forme galénique de la composition selon l'invention peut également être à libération immédiate : une telle forme galénique permet une absorption rapide du précurseur de NAD et ainsi un délai d'action réduit. Les formes galéniques à libération immédiate sont notamment les comprimés dispersibles, orodispersibles, effervescents et les lyophilisats oraux.

La forme galénique de la composition selon l'invention peut également être à libération retardée. La dissolution et l'absorption du précurseur de NAD s'effectuent au niveau intestinal, ce qui limite l'irritation gastrique ou la dégradation des principes actifs fragiles à pH acide. Il s'agit majoritairement de formes gastrorésistantes, c'est-à-dire que les comprimés ou granulés sont recouverts d'un film polymérique, insoluble en milieu acide mais perméable à l'eau en milieu alcalin ou de type lipidique dégradé par les lipases intestinales.

La forme galénique de la composition selon l'invention peut également être à libération prolongée et séquentielle. Les formes à libération séquentielle (libération à intervalle de temps précis) et prolongée (libération continue du principe actif jusqu'à épuisement) favorisent l'étalement de la libération du principe actif dans le temps afin de maintenir une concentration plasmatique efficace plus longtemps dans l'organisme du patient. De telles formes galéniques permettent d'obtenir un soulagement de la douleur du patient pendant un laps de temps plus long, et d'espacer les prises de médicament.

Le mode d'administration et la forme galénique sont déterminés par l'homme du métier en fonction de la localisation anatomique de la douleur à traiter et du patient. Il est fait référence à la dernière édition de Remington's Pharmaceutical Sciences.

Les compositions selon l'invention peuvent être formulées avec des supports, des excipients et des diluants qui conviennent en soi pour ces formulations, tels que le lactose, le dextrose, le saccharose, le sorbitol, le mannitol, les amidons, la gomme d'acacia, le phosphate de calcium, les alginates, la gomme adragante, la gélatine, le silicate de calcium, la cellulose microcristalline, polyvinylpyrrolidone, polyéthylène glycol, cellulose, eau (stérile), méthylcellulose, hydroxybenzoates de méthyle et de propyle, talc, stéarate de magnésium, huiles alimentaires, huiles végétales et minérales ou mélanges appropriés de celles-ci. Les formulations peuvent éventuellement contenir d'autres substances qui sont couramment utilisées dans les formulations pharmaceutiques, telles que des agents lubrifiants, des agents mouillants, des agents émulsifiants et de suspension, des agents dispersants, des désintégrants, des agents de charge, des charges, des agents de conservation, des édulcorants, des aromatisants, des régulateurs de débit, des agents de démoulage, etc. Les compositions peuvent également être formulées de manière à permettre une libération rapide, prolongée ou retardée du ou des composés actifs qu'elles contiennent.

Les compositions selon l'invention sont de préférence sous forme de doses unitaires et peuvent être conditionnées de manière appropriée, par exemple dans une boîte, un blister, un flacon, une bouteille, un sachet, une ampoule ou dans tout autre support ou récipient approprié à dose unique ou à doses multiples (qui peut être correctement étiqueté) ; optionnellement avec une ou plusieurs notice(s) contenant des informations sur le produit et/ou des instructions d'utilisation.

### Combinaisons thérapeutiques

Avantageusement, le NMN, un de ses dérivés pharmaceutiquement acceptables ou un de ses sels pharmaceutiquement acceptables, et la composition selon l'invention sont utilisés en combinaison avec au moins un agent thérapeutique supplémentaire. Dans un mode de réalisation, la composition selon l'invention comprend le NMN un dérivé pharmaceutiquement acceptable ou un sel pharmaceutiquement acceptable, un excipient pharmaceutiquement acceptable et au moins un agent thérapeutique supplémentaire.

Avantageusement, l'au moins un agent thérapeutique supplémentaire est choisi parmi les antibiotiques, les antifongiques, les antiviraux et leurs combinaisons. Ainsi, l'utilisation de NMN, un de ses dérivés pharmaceutiquement acceptables ou un de ses sels pharmaceutiquement acceptables, ou de la composition selon l'invention permet de réduire la douleur nociceptive liée à une infection bactérienne, fongique, virale ou leurs combinaisons. Par exemple, le NMN, un de ses précurseurs pharmaceutiquement acceptables, un de ses dérivés pharmaceutiquement acceptables ou un de ses sels pharmaceutiquement acceptables, ou la composition selon l'invention peut être utilisé en combinaison avec un antibiotique. De telles combinaisons peuvent trouver leur utilité pour des applications topiques, par exemples pour des pastilles pour la gorge permettant d'une part de réduire la charge microbienne en cas d'angine bactérienne et de soulager la douleur liée à l'inflammation locale. Le NMN, un de ses précurseurs pharmaceutiquement acceptables, un de ses dérivés pharmaceutiquement acceptables ou un de ses sels pharmaceutiquement acceptables, ou la composition selon l'invention peuvent également être utilisés en combinaison avec un antifongique. De telles combinaisons peuvent par exemple trouver leur utilité pour traiter ou prévenir des mycoses urinaires, vaginales, cutanées etc.

Dans un mode de réalisation, l'antibiotique peut être choisi parmi les beta-lactamines, les cyclines, les macrolides, les macrolides apparentés, les quinolones, les fluoroquinolones, les quinoléines, les aminosides, les acides fusidiques, les lincosamides, les phénicolés, les polymyxines, les sulfamides associés ou non au triméthoprime, les antilépreux, la fosfomycine, la mupirocine le nitrofurantoïne, les nitrofuranes, les nitro-imidazoles, les oxazolidinones, les glycopeptides, les lipopeptides, les polymixines et les antituberculeux.

Dans un mode de réalisation, l'antifongique peut être choisi parmi l'amphotéricine B, la flucytosine, les dérivés azolés, la terbinafine, le sulfure de sélénium, le trichlocarban, la nystatine, la griséofulvine, l'amorolfine, l'acide salicylique, la ciclopiroxolamine, l'amorolfine, le tolfanate et leurs combinaisons.

Les dérivés azolés antifongiques peuvent notamment être choisis parmi le bifonazole, le clotrimazole, l'éconazole, le fenticonazole, le fluconazole, l'isoconazole, l'itroconazole, le kétoconazole, le miconazole, l'omoconazole, l'oxiconazole, le sertaconazole, le sulconazole, le tioconazole, le voriconazole et leurs combinaisons.

Les antiviraux peuvent notamment être choisis parmi les antagonistes du récepteur CCR5, les antiviraux systémiques, les dérivés de l'acide phosphorique, les inhibiteurs d'intégrase, les inhibiteurs de fusion, les inhibiteurs de la neuraminidase, les inhibiteurs de la transcriptase inverse non nucléoside, les inhibiteurs de la transcriptase inverse nucléoside, les inhibiteurs de protéase, les nucléosides et nucléotides, inhibiteurs de la transcriptase inverse exclus et leurs combinaisons.

Dans un mode de réalisation, la composition selon l'invention comprend en outre au moins un antalgique. L'antalgique peut appartenir au niveau I, au niveau II, au niveau III selon la classification de l'OMS ou leurs combinaisons.

Dans un mode de réalisation, l'antalgique de niveau I est choisi parmi le paracétamol, l'aspirine, les anti-inflammatoires non stéroïdiens, les dérivés de la cortisone et leurs combinaisons.

L'anti-inflammatoire non stéroïdien peut être choisi parmi l'ibuprofène, le kétoprofène, le naproxène, l'alminoprofène l'acéclofénac, l'acide méfénamique, l'acide niflumique, l'acide tiaprofénique, le célécoxib, le dexkétoprofène, le diclofénac, l'étodolac, l'étoricoxib, le fénoprofène, le flurbiprofène, l'indométacine, le méloxicam, le nabumétone, le piroxicam, le sulindac, le ténoxicam et leurs combinaisons.

Le dérivé de la cortisone peut être choisi parmi la bétaméthasone, la ciprofloxacine, le cortivazol, la dexaméthasone, la fludrocortisone, le methylprednisolone, le prednisolone et la triamcinolone

L'antalgique de niveau II peut être choisi parmi la codéine, la dihydrocodéine, le tramadol et leurs combinaisons.

L'antalgique de niveau III peut être est choisi parmi la morphine, la buprénorphine, le fentanyl, l'hydromorphone, la nalbuphine, l'oxycodone, la péthidine et leurs combinaisons.

Dans le contexte de la présente invention, un « excipient » désigne toute substance autre que le NMN dans la composition et n'ayant pas d'effet thérapeutique. L'excipient n'interagit pas sur le plan chimique avec le NMN ou tout autre agent thérapeutique supplémentaire.

L'excipient peut être choisi parmi un agent de charge, un lubrifiant, un arôme, un colorant, un émulsifiant, un agent de compression, un diluant, un conservateur, un gélifiant, un plastifiant, un tensioactif ou leurs combinaisons. L'homme de l'art sait quel excipient choisir en fonction de la forme galénique qu'il aura choisie.

La composition selon l'invention peut être une composition pharmaceutique. En ce cas, l'excipient est un excipient pharmaceutiquement acceptable tel que défini ci-dessus.

La composition selon l'invention peut également être un complément alimentaire.

### Dérivés et précurseurs du NMN

Selon l'invention, le dérivé du NMN peut être choisi parmi l'alpha nicotinamide mononucléotide (α-NMN), le dihydronicotinamide mononucléotide (noté NMN-H), le composé de formule (I): 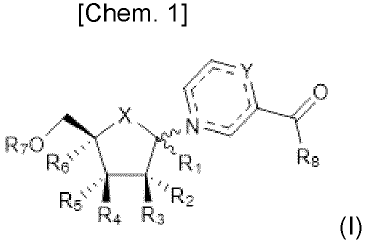 ou un de ses stéréoisomères, un de ses sels, un de ses hydrates, un de ses solvates ou un de ses cristaux pharmaceutiquement acceptable de celui-ci, dans lequel :
- X est choisi parmi O, CH₂, S, Se, CHF, CF₂ et C=CH₂;
- R₁ est choisi parmi H, azido, cyano, alkyle en C₁-C₈, thio-alkyle en C₁-C₈, hétéroalkyle en C₁-C₈ et OR ; dans lequel R est choisi parmi H et alkyle en C₁-C₈ ;
- R₂, R₃, R₄ et R₅ sont choisis indépendamment l'un de l'autre parmi H, halogène, azido, cyano, hydroxyle, alkyle en C₁-C₁₂, thio-alkyle en C₁-C₁₂, hétéroalkyle en C₁-C₁₂, haloalkyle en C₁-C₁₂ et OR ; dans lequel R est choisi parmi H, alkyle en C₁-C₁₂, C(O)(C₁-C₁₂)alkyle, C(O)NH(C₁-C₁₂)alkyle, C(O)O(C₁-C₁₂)alkyle, C(O)aryle, C(O)(C₁-C₁₂)alkyle aryle, C(O)NH(C₁-C₁₂)alkyle aryle, C(O)O(C₁-C₁₂)alkyle aryle et C(O)CHR_{AA}NH₂ ; dans lequel R_{AA} est une chaîne latérale choisie parmi un acide aminé protéinogène ;
- R₆ est choisi parmi H, azido, cyano, C₁-C₈ alkyle, C₁-C₈ thio-alkyle, C₁-C₈ hétéroalkyle et OR ; dans lequel R est choisi parmi H et C₁-C₈ alkyle;
- R₇ est choisi parmi H, P(O)R₉R₁₀ et P(S)R₉R₁₀ ; dans lequel
- R₉ et R₁₀ sont choisis indépendamment l'un de l'autre parmi OH, OR₁₁, NHR₁₃, NR₁₃R₁₄, un alkyle en C₁-C₈, un alcényle en C₂-C₈, un alcynyl en C₂-C₈, un cycloalkyl en C₃-C₁₀, un aryle en C₅-C₁₂, (C₁-C₈)alkyle aryle, (C₁-C₈)aryle alkyle, (C₁-C₈) heteroalkyle, (C₁-C₈) heterocycloalkyle, un heteroaryle et NHCHR_{A}R_{A'}C(O)R₁₂ ; dans lequel :
- R₁₁ est choisi parmi un groupe C₁-C₁₀ alkyle, C₃-C₁₀ cycloalkyle, C₅-C₁₈ aryle, C₁-C₁₀ alkylaryle, C₅-C₁₂ aryle substitué, C₁-C₁₀ heteroalkyle, C₃₋C₁₀ heterocycloalkyle, C₁-C₁₀ haloalkyle, un heteroaryle, -(CH₂),C(O)(C₁-C₁₅)alkyle, -(CH₂)ₙOC(O)(C₁-C₁₅)alkyle, -(CH₂)ₙOC(O)O(C₁-C₁₅)alkyle, -(CH₂)ₙSC(O)(C₁-C₁₅)alkyle, - (CH₂)ₙC(O)O(C₁-C₁₅)alkyle and -(CH₂)ₙC(O)O(C₁-C₁₅)alkyle aryle; dans lesquels n est un entier choisi de 1 à 8; P(O)(OH)OP(O)(OH)₂, halogène, nitro, cyano, C₁-C₆ alkoxy, C₁-C₆ haloalkoxy, -N(R₁₁ₐ)₂, C₁-C₆ acylamino, -COR_{11b}, -O COR_{11b} ; NHSO₂(C₁-C₆ alkyl), - SO₂N(R₁₁ₐ)₂ SO₂ dans lequel chacun de R₁₁ₐ est indépendamment choisi parmi H et un C₁-C₆alkyle et R_{11b} est indépendamment choisi parmi OH, C₁-C₆ alkoxy, NH₂, NH(C₁-C₆ alkyle) ou N(C₁-C₆ alkyle)₂ ;;
- R₁₂ est choisi parmi H, C₁₋C₁₀ alkyle, C₂-C₈ alcényle, C₂-C₈ alcynyle, C₁₋C₁₀ haloalkyle, C₃₋C₁₀ cycloalkyle, C₃₋C₁₀ heterocycloalkyle, C₅-C₁₈ aryle, C₁-C₄ alkylaryle and C₅-C₁₂ heteroaryle; dans lesquels lesdits groupes aryle ou heteroaryle sont optionnellement substitué parmi un ou deux groupes choisi parmi un halogène, trifluoromethyl, C₁-C₆ alkyle, C₁-C₆ alkoxy et cyano; et
- R_{A} et R_{A'} sont indépendamment choisi parmi H, un C₁₋C₁₀ alkyle, C₂-C₁₀ alcényle, C₂-C₁₀ alcynyle, C₃₋C₁₀ cycloalkyle, C₁-C₁₀ thio-alkyle, C₁₋C₁₀ hydroxylalkyle, C₁-C₁₀ alkylaryle and C₅-C₁₂ aryle, C₃₋C₁₀ heterocycloalkyle, un heteroaryle, - (CH₂)₃NHC(=NH)NH₂, (1H-indol-3-yl)methyl, (1H-imidazol-4-yl)methyl et une chaine latérale choisie parmi un acide aminé protéinogène ou un acide aminé non protéinogène; dans lesquels lesdits groupes aryle sont optionnellement substitué avec un groupe choisi parmi un hydroxyle, C₁₋C₁₀ alkyl, C₁-C₆ alkoxy, un halogène, un nitro et un cyano; ou
- R₉ et R₁₀ forment ensemble avec les atomes de phosphore auxquels ils sont attaché un cycle à 6 membres dans lequel -R₉-R₁₀- représente -CH₂-CH₂-CHR-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un C₁-C₆ alkyle, un C₁-C₆ alkoxy et cyano; ou
   R₉ et R₁₀ forment ensemble avec les atomes de phosphore auxquels ils sont attaché un cycle à 6 membres dans lequel -R₉-R₁₀- représente -O-CH₂-CH₂-CHR-O-; dans lequel R est choisi parmi H, un groupe (C₅-C₆) aryle et (C₅-C₆) heteroaryle, dans lequel lesdits groupes aryle ou heteroaryle sont optionnellement substitués par un halogène, trifluoromethyl, un (C₁-C₆) alkyle, un (C₁-C₆) alkoxy et cyano;
- R₈ est choisi parmi H, OR, NHR₁₃, NR₁₃R₁₄, NH-NHR₁₃, SH, CN, N₃ et halogène ; dans lequel R₁₃ et R₁₄ sont choisis indépendamment l'un de l'autre parmi H, (C₁-C₈) alkyle et (C₁-C₈) alkyle aryle, et -CR_{B}R_{C}-C(O)-OR_{D} dans lequel R_{B} et Rc sont indépendamment un atome d'hydrogène, un (C₁-C₆) alkyle, un (C₁-C₆) alkoxy, benzyle, indolyle ou imidazolyle, où le (C₁-C₆) alkyle et le (C₁-C₆)) alkoxy peuvent être éventuellement et indépendamment l'un de l'autre substitué par un ou plusieurs des groupes halogène, amino, amido, guanidyle, hydroxyle, thiol ou carboxyle, et le groupe benzyle est éventuellement substitué par un ou plusieurs des groupes halogène ou hydroxyle, ou R_{B} et Reforment ensemble avec l'atome de carbone auquel ils sont attachés un groupe cycloalkyle en C₃-C₆ éventuellement substitué par un ou plusieurs halogènes, amino, amido, guanidyle, hydroxyle, thiol et carboxyle et R_{D} est un hydrogène, un (C₁-C₆) alkyle, un (C₂-C₆) alcényle, un (C₂-C₆) alcynyle ou un (C₃-C₆) cycloalkyle ;
- Y est choisi parmi CH, CH₂, C(CH₃)₂ et CCH₃ ;
- 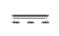 représente une simple ou une double liaison selon Y ; et
- 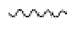 représente l'anomère alpha ou bêta selon la position de R₁
   ou un de ses stéréoisomères, un de ses sels, un de ses hydrates, un de ses solvates ou un de ses cristaux
      ou
      le composé de formule (II) : 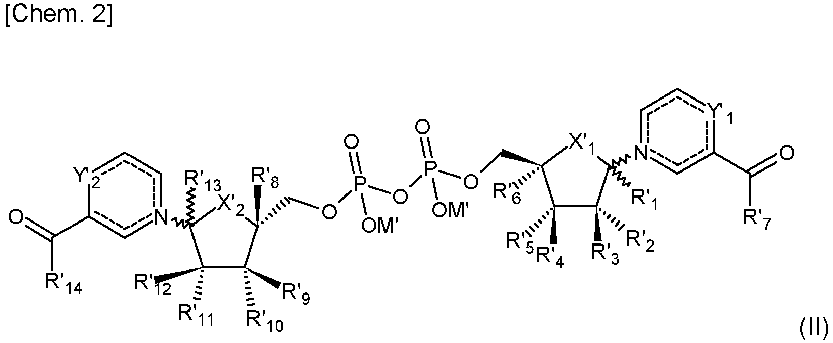
   ou un de ses stéréoisomères, un de ses sels, un de ses hydrates, un de ses solvates ou un de ses cristaux, dans lequel
      - X'₁ et X'₂ sont indépendamment choisis parmi O, CH₂, S, Se, CHF, CF₂, et C=CH₂ ;
      - R'₁ et R'13 sont indépendamment choisis parmi H, azido, cyano, un alkyl en C1-C8, un thio-alkyl en C1-C8, un hétéroalkyl en C1-C8, et OR, dans lequel R est choisi parmi H et un alkyl en C1-C8,
      - R'₂, R'₃, R'₄, R'₅, R'₉, R'₁₀, R'₁₁, R'₁₂ sont indépendamment choisis parmi H, un halogène, un azido, un cyano, un hydroxyl, un alkyl en C₁-C₁₂, un thioalkyl en C₁-C₁₂, un hétéro-alkyl en C₁-C₁₂, un haloalkyl en C₁-C₁₂ et OR, dans lequel R peut être choisi parmi H, un alkyl en C₁-C₁₂, un C(O)(C₁-C₁₂) alkyle, un C(O)NH(C₁-C₁₂) alkyle, un C(O)O(C₁-C₁₂) alkyle, un C(O) aryle, un C(O)(C₁-C₁₂) aryle, un C(O)NH(C₁-C₁₂) alkyle aryle, un C(O)O(C₁-C₁₂) alkyle aryle ou un groupe C(O)CHR_{AA}NH₂ dans lequel R_{AA} est une chaine latérale choisi parmi un acide aminé protéogène ;
      - R'₆ et R'₈ sont indépendamment choisis parmi H, un azido, un cyano, un alkyl en C₁-C₈ et OR, dans lequel R est choisi parmi H et un alkyl en C₁-C₈ ;
      - R'₇ et R'₁₄ sont indépendamment choisis parmi H, OR, NHR, NRR', NH-NHR, SH, CN, N₃ et un halogène, dans lequel R et R' sont indépendamment choisit parmi H et un (C₁-C₈ ) alkyle aryle ;
      - Y'1 et Y'2 sont indépendamment choisis parmi CH, CH₂, C(CH₃)₂ ou CCH₃ ;
      - M' est choisi parmi H ou un contre-ion adapté ;
      - 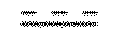 représente une liaison simple ou double en fonction de Y'1 et Y'2 ; et
      - 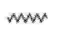 représente un anomère alpha ou bêta en fonction de la position de R'₁ et R'₁₃ ;
   et leurs combinaisons.
A noter que seuls les composés de formule (I) et (II) revendiqués font parties de l'invention.

Dans un premier mode de réalisation non-revendiqué, le dérivé pharmaceutiquement acceptable est le composé de formule (I).

Dans une variante du premier mode de réalisation, X représente un oxygène.

Dans une variante du premier mode de réalisation, R₁ et R₆ représentent chacun indépendamment l'un de l'autre un hydrogène.

Dans une variante du premier mode de réalisation, R₂, R₃, R₄ et R₅ représentent chacun indépendamment l'un de l'autre un hydrogène ou un OH.

Dans une variante du premier mode de réalisation, Y représente un CH.

Dans une variante du premier mode de réalisation, Y représente un CH₂.

Dans une variante du premier mode de réalisation, R₇ représente un hydrogène.

Dans une variante du premier mode de réalisation, R₇ représente P(O)(OH)₂.

Dans une variante du premier mode de réalisation, le composé de l'invention est choisi parmi les composés de formule I-A à I-H :

**[Tableau 1]**

| Composés n° (anomères) | Structure |
|---|---|
| I-A (beta) | 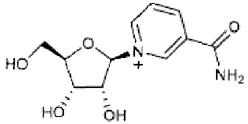 |
| I-B (alpha) | 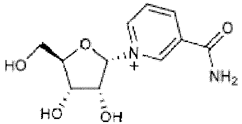 |
| I-C (beta) | 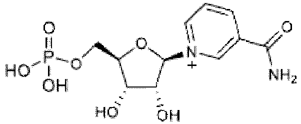 |
| I-D (alpha) | 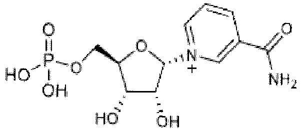 |
| I-E (beta) | 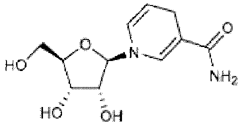 |
| I-F (alpha) | 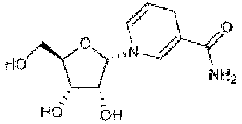 |
| I-G (bêta) | 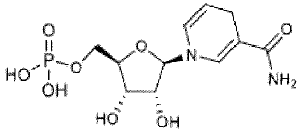 |
| I-H (alpha) | 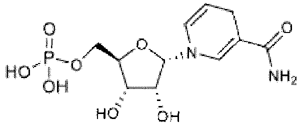 |

| | |
|---|---|
| Seuls les composés I-C, I-D, I-G et I-H font parties de l'invention. | |

Dans une variant préférée de l'invention, le dérivé pharmaceutiquement acceptable est l'alpha-NMN de formule :

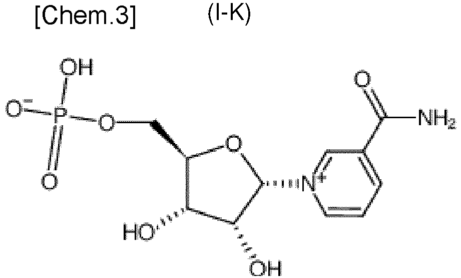

Dans un deuxième mode de réalisation non-revendiqué, le dérivé pharmaceutiquement acceptable est le composé de formule (II).

Dans une variante du deuxième mode de réalisation, X'1 et X'2 représentent chacun indépendamment un oxygène.

Dans une variante du deuxième mode de réalisation, R'7 et R'14 représentent chacun indépendamment un NH₂.

Dans une variante du deuxième mode de réalisation, R'1 et/ou R'13 représentent chacun indépendamment un hydrogène.

Dans une variante du deuxième mode de réalisation, R'6 et/ou R'8 représentent chacun indépendamment un hydrogène.

Dans une variante du deuxième mode de réalisation, R'2, R'3, R'4, R'5, R'9, R'10, R'11 et R'12 représentent chacun indépendamment un hydrogène.

Dans une variante du deuxième mode de réalisation, R'2, R'3, R'4, R'5, R'9, R'10, R'11 et R'12 représentent chacun indépendamment un OH.

Dans une variante du deuxième mode de réalisation, Y'1 et Y'2 représentent chacun indépendamment un CH.

Dans une variante du deuxième mode de réalisation, Y'1 et Y'2 représentent chacun indépendamment un CH2.

Le composé selon l'invention est choisi parmi les composés de formule II-A à II-F :

**[Tableau 2]**

| Composé n° (anomère) | Structure |
|---|---|
| II-A (bêta, bêta) | 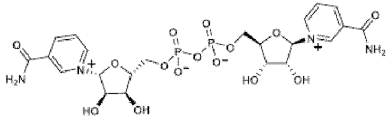 |
| II-B (bêta, alpha) | 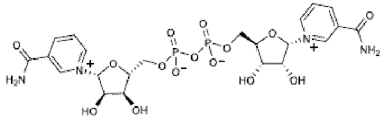 |
| II-C (alpha, alpha) | 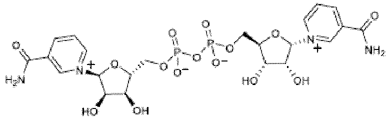 |
| II-D (bêta, bêta) | 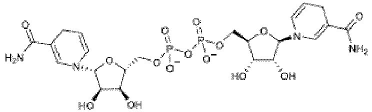 |
| II-E (bêta, alpha) | 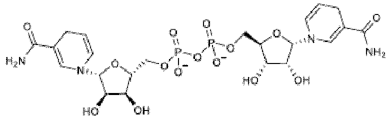 |
| II-F (alpha, alpha) | 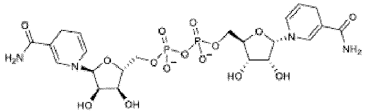 |

Dans un quatrième mode de réalisation préféré, le dérivé pharmaceutiquement acceptable est le NMN-H :

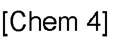

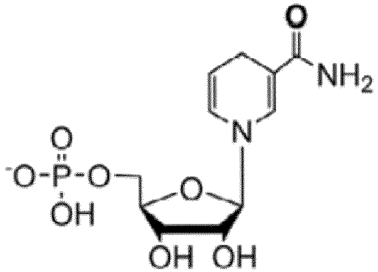

Le précurseur pharmaceutiquement acceptable non-revendiqué, est le nicotinamide riboside (noté NR) : 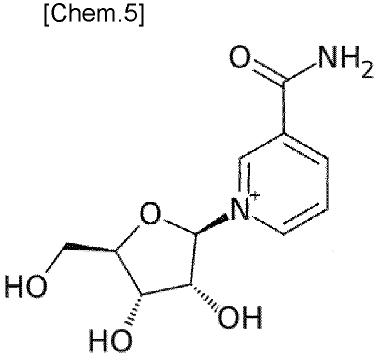 ou le dihydronicotinamide riboside (noté -NR-H) de formule : 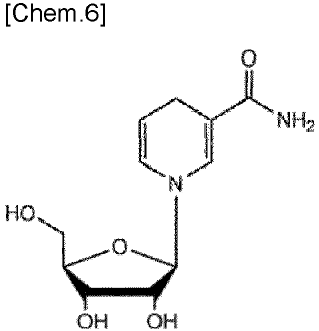 ou leur combinaison. De préférence, le précurseur non-revendiqué, est le nicotinamide riboside (NR)

De préférence, le dérivé du NMN est le dihydronicotinamide mononucléotide (NMN-H) et/ou l'alpha-NMN.

### Procédé de préparation des composés de formule (I) et (II)

Les dérivés de formule (I) ou de formule (II) peuvent être préparés selon toute méthode bien connue de l'homme du métier.

Procédé de préparation des composés de formule (I)

Les dérivés de formule (I) peuvent être préparés selon le procédé décrit dans la demande internationale WO 2017/024255A1.

Notamment, les dérivés de formule (I) ainsi que l'alpha-NMN peuvent être préparés selon la méthode décrite ci-dessous.

En particulier, les composés de formule (I) divulgués ici peuvent être préparés comme décrit ci-dessous à partir des substrats A-E. Il sera entendu par un homme du métier que ces schémas réactionnels ne sont en aucune façon limitatifs et que des variations peuvent être faites sans s'écarter de l'esprit et de la portée de la présente invention.

Selon un mode de réalisation, l'invention concerne une méthode de préparation des composés de formule (I) tels que décrits ci-dessus.

La méthode implique dans une première étape la mono-phosphorylation d'un composé de formule (A), en présence de chlorure de phosphoryle et d'un phosphate de trialkyle, pour conduire au phosphorodichloridate de formule (B), 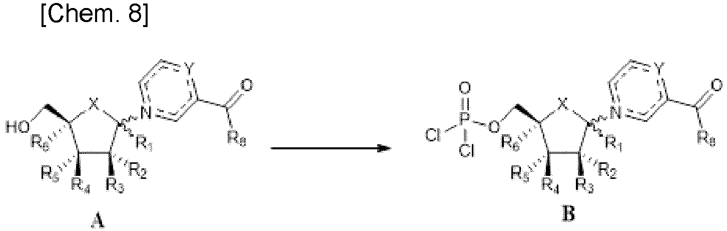 dans lesquels X, R₁, R₂, R₃, R₄, R₅, R₆, R₈, Y, 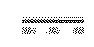 et 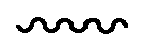 sont tels que définis ci-dessus pour les composés de formule (I).

Dans une seconde étape, le phosphorodichloridate de formule (B) est hydrolysé pour conduire au phosphate de formule (C), 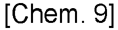 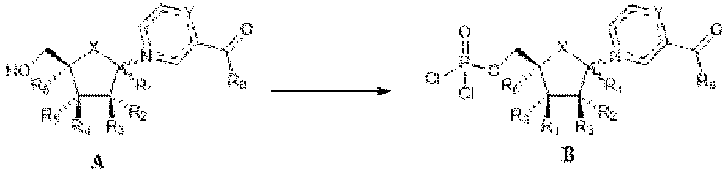 dans lesquels X, R₁, R₂, R₃, R₄, R₅, R₆, R₈, Y, 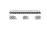 et 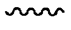 sont tels que définis ci-dessus pour les composés de formule (I).

Selon un mode de réalisation, le composé de formule (A) est synthétisé à l'aide de diverses méthodes connues de l'homme du métier.

Selon un mode de réalisation, le composé de formule (A) est synthétisé par réaction du pentose de formule (D) avec un dérivé azoté de formule (E), dans lequel R, R₂, R₃, R₄, R₅, R₆, R₇, Y sont tels que décrits ci-dessus pour les composés de formule I, conduisant au composé de formule (A-1) qui est ensuite déprotégé sélectivement pour donner le composé de formule (A), 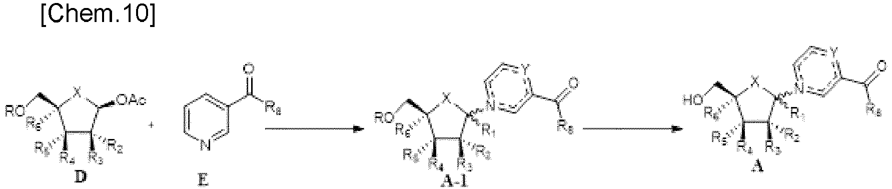 dans lesquels X, R₁, R₂, R₃, R₄, R₅, R₆, R₈, Y, 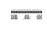 et 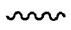 sont tels que définis ci-dessus pour les composés de formule (I).

Selon un mode de réalisation, R est un groupe protecteur approprié connu de l'homme du métier. Dans un mode de réalisation, le groupe protecteur est choisi parmi les triarylméthyles et/ou silyles. Des exemples non limitatifs de triarylméthyle comprennent les groupes trityle, monométhoxytrityle, 4,4'-diméthoxytrityle et 4,4',4"-triméthoxytrityle. Des exemples non limitatifs de groupes silyle comprennent les groupes triméthylsilyle, tert-butyldiméthylsilyle, triisopropylsilyle, tert-butyldiphénylsilyle, tri-iso-propylsilyloxyméthyle et [2-(triméthylsilyl)éthoxy]méthyle.

Selon un mode de réalisation, tout groupe hydroxyle attaché au pentose est protégé par un groupe protecteur approprié connu de l'homme du métier.

Le choix et l'échange des groupes protecteurs relèvent de la compétence de l'homme du métier. Les groupes protecteurs peuvent également être éliminés par des méthodes bien connues de l'homme du métier, par exemple, avec un acide (par exemple, un acide minéral ou organique), une base ou une source de fluorure.

Dans un mode de réalisation préféré, le dérivé azoté de formule (E) est couplé au pentose de formule (D) par une réaction en présence d'un acide de Lewis conduisant au composé de formule (A-1). Des exemples non limitatifs d'acides de Lewis comprennent le TMSOTf, BF₃.OEt₂, TiCl₄ et FeCl₃.

Dans un mode de réalisation, la méthode de la présente invention comprend en outre une étape de réduction du composé de formule (A) par diverses méthodes bien connues de l'homme du métier conduisant au composé de formule (A') dans lequel est CH₂ et R₁, R₂, R₃, R₄, R₅, R₆, R₈, Y, 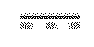 et 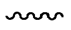 sont tels que définis ci-dessus pour les composés de formule (I).

Dans un mode de réalisation particulier, la présente invention concerne une méthode de préparation des composés de formule I-A, I-C, I-E, I-G.

Dans une première étape, le nicotinamide de formule E est couplé au ribose tétraacétate de formule D par une réaction de couplage en présence d'un acide de Lewis, conduisant au composé de formule A-1 :

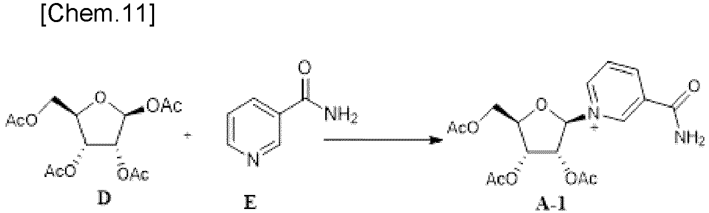

Dans une seconde étape, un traitement ammoniacal du composé de formule A-1 est réalisé, conduisant au composé de formule I-A :

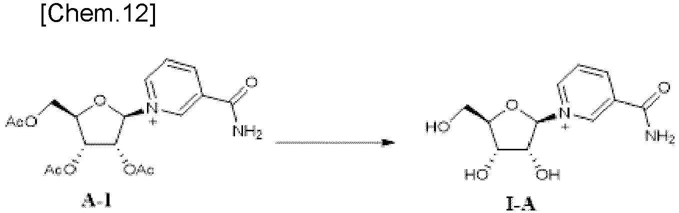

Dans une troisième étape, la mono-phosphorylation du composé de formule I-A, en présence de chlorure de phosphoryle et d'un phosphate de trialkyle, conduit au phosphorodichloridate de formule I-A' :

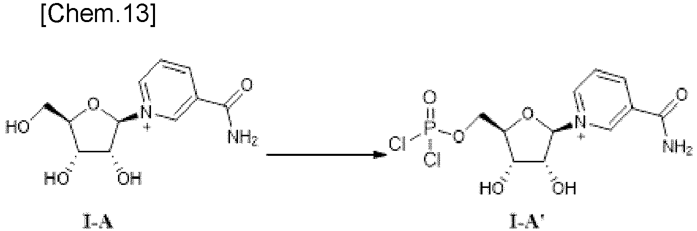

Dans une quatrième étape, le phosphorodichloridate de formule B est hydrolysé pour conduire au composé de formule I-C :

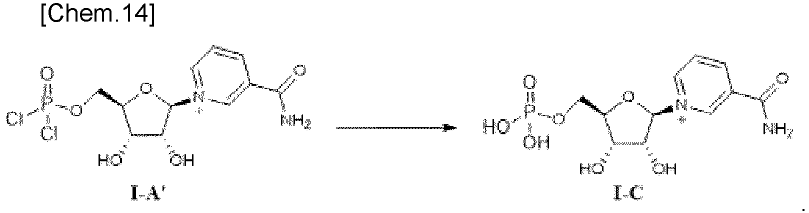

Dans un mode de réalisation, une étape de réduction du composé de formule I-A est réalisée, conduisant au composé de formule I-E.

Le composé de formule I-E est ensuite mono-phosphorylé comme décrit à la quatrième étape et hydrolysé pour conduire au composé de formule I-G.

Selon un mode de réalisation, les composés de formule (I) sont sélectionnés parmi les composés I-A à I-H du tableau ci-dessous :

**[Tableau 1]**

| Composés n° (anomères) | Structure |
|---|---|
| I-A (beta) | 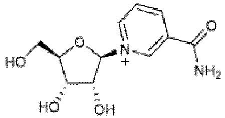 |
| I-B (alpha) | 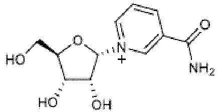 |
| I-C (beta) | 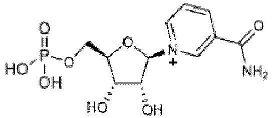 |
| I-D (alpha) | 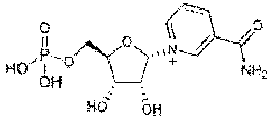 |
| I-E (beta) | 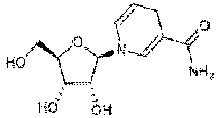 |
| 1-F (alpha) | 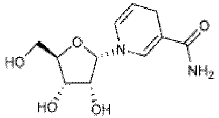 |
| I-G (beta) | 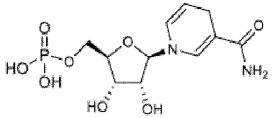 |
| I-H (alpha) | 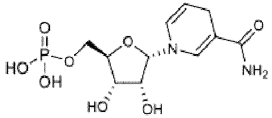 |

Seuls les composés I-C, I-D, I-G, I-H font parties de l'invention.

Dans un mode de réalisation préféré, les composés de l'invention sont les composés de formule I-C, et I-G du tableau ci-dessus ou un sel et/ou solvate pharmaceutiquement acceptable de ceux-ci. Dans un mode de réalisation encore plus préféré, le composé est le composé de formule I-C ou I-D un sel et/ou solvate pharmaceutiquement acceptable de celui-ci.

Procédé de préparation des dérivés de formule (II)

En particulier, les composés de formule II présentés ici peuvent être préparés comme décrit ci-dessous à partir des substrats X-XIII.

Selon un mode de réalisation, l'invention porte sur une méthode de préparation du composé de formule I décrit ci-dessus.

La méthode consiste d'abord à mono-phosphoryler un composé de formule X, en présence de chlorure de phosphoryle dans un phosphate de trialkyle, pour obtenir le composé phosphorodichloridate XI, 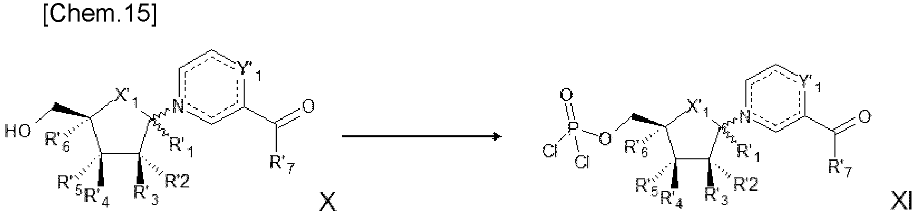 dans laquelle X'₁, R'₁, R'₂, R'₃, R'₄, R'₅, R'₆, R'₇, Y'₁, 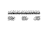 et 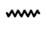 sont tels que définis ci-dessus.

Dans une deuxième étape, l'hydrolyse du phosphorodichloridate XI obtenu dans la première étape donne le composé de phosphate de formule XII, 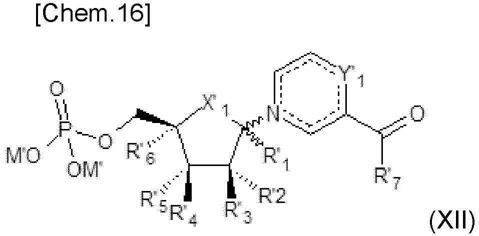 dans laquelle X'₁, R'₁, R'₂, R'₃, R'₄, R'₅, R'₆, R'₇, Y'₁,M', 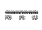 et 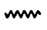 sont tels que définis ci-dessus.

Le composé de phosphate de formule XII obtenu dans la deuxième étape est ensuite mis à réagir avec un composé de phophorodichloridate de formule XIII obtenu comme décrit dans la première étape, 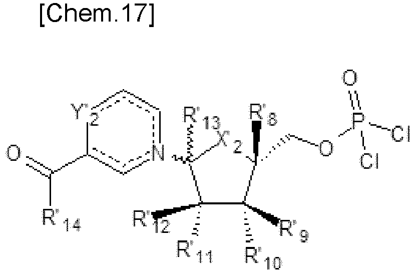 dans laquelle X'₂, R'₈, R'₉, R'₁₀, R'₁₁, R'₁₂, R'₁₃, R'₁₄, Y'₂, 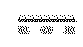 et 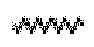 sont tels que décrits ici pour la formule II, pour donner le composé de formule II tel que décrit ici.

Selon un mode de réalisation, le procédé comprend en outre une étape de réduction du composé de formule II, en utilisant diverses méthodes connues des spécialistes, pour donner le composé de formule II, où Y'₁ et Y'₂ sont identiques et représentent chacun CH₂ et où X'₁, X'₂, R'₁, R'₂, R'₃, R'₄, R'₅, R'₆, R'₇, R'₈, R'₉, R'₁₀, R'₁₁, R'₁₂, R'₁₃, R'₁₄, Y'₁, Y'₂ et 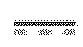 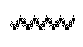 sont tels que décrits ici pour la formule II.

Dans une variante, R est un groupe de protection approprié connu de ceux qui sont habiles dans cet art. Les groupes triarylméthyle et/ou silyle sont des exemples de groupes protecteurs appropriés. Les exemples non limitatifs de triarylméthyle comprennent le trityle, le monométhoxytrityle, le 4,4'-diméthoxytrityle et le 4,4',4"-triméthoxytrityle. Les exemples non limitatifs de groupes silyle comprennent le triméthylsilyle, le tert-butyldiméthylsilyle, le triisopropylsilyle, le tert-butyldiphénylsilyle, le tri-iso-propylsilyloxyméthyle et le [2-(triméthylsilyl)éthoxy]méthyle.

Selon l'une des représentations, tout groupe hydroxy attaché à l'anneau pentose est protégé par un groupe de protection approprié connu des spécialistes de cet art.

La sélection et l'échange des groupes de protection relèvent de la compétence de ceux qui sont compétents dans ce domaine. Tout groupe de protection peut également être éliminé par des méthodes connues dans l'art, par exemple, avec un acide (par exemple, un minéral ou un acide organique), une base ou une source de fluorure.

Selon un mode de réalisation préféré, les dérivés azotés de formule XV sont ajoutés au pentose XIV par une réaction de couplage en présence d'un acide de Lewis pour donner le composé de formule X-1. Parmi les exemples non limitatifs d'acides de Lewis appropriés, on peut citer le TMSOTf, le BF3.OEt2, le TiCl4 et le FeCl3.

Selon une réalisation spécifique, l'invention porte sur une méthode de préparation du composé de formule VIII, 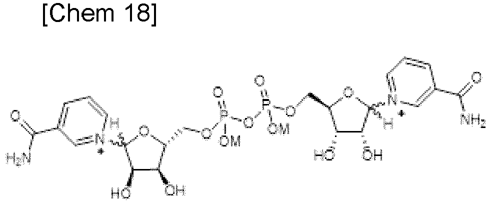 ou leurs sels et/ou solvates pharmaceutiquement acceptables.

Dans une première étape, le nicotinamide de formule XV est ajouté au tétraacétate de ribose XIV, par une réaction de couplage en présence d'un acide de Lewis, pour donner le composé de formule X-1 :

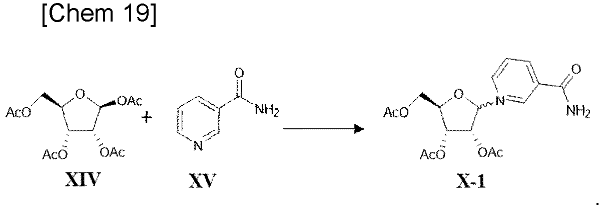

Dans une deuxième étape, un traitement ammoniacal du composé de formule X-1 donne le composé de formule X :

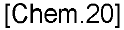

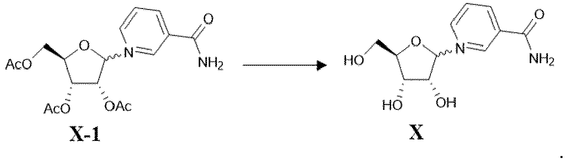

Dans une troisième étape, la mono-phosphorylation d'un composé de formule X, en présence de chlorure de phosphoryle dans un phosphate de trialkyle, donne le composé phosphorodichloridate XI :

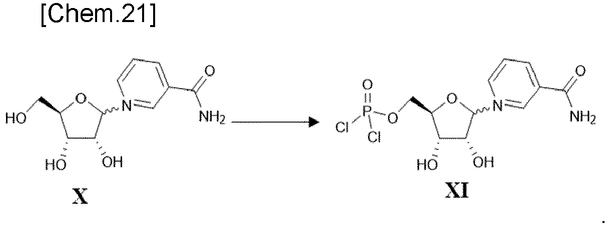

Dans une quatrième étape, le composé phosphorodichloridate XI obtenu dans la troisième étape est partiellement hydrolysé pour donner le composé phosphate de formule XII :

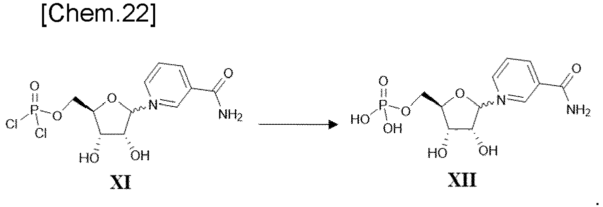

Dans une cinquième étape, on fait ensuite réagir le composé phosphate de formule XII obtenu dans la quatrième étape, avec le composé phosphorodichloridate de formule XI obtenu comme décrit dans la troisième étape, pour obtenir le composé de formule VIII.

Selon une autre concrétisation spécifique, l'invention porte sur une méthode de préparation du composé de formule IX, 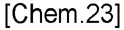 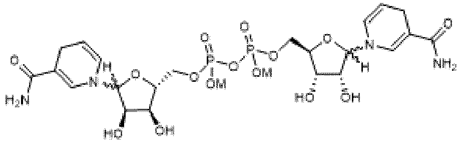 ou leurs sels et/ou solvates pharmaceutiquement acceptables.

D'après une variante, le composé de formule IX est obtenu à partir du composé de formule VIII, préalablement synthétisé comme décrit ci-dessus.

Dans cette réalisation, le composé de formule IX est obtenu en réduisant le composé de formule VIII, à l'aide d'un agent réducteur approprié connu des spécialistes, pour donner le composé de formule IX.

Selon un mode de réalisation, les composés préférés de l'invention sont les composés II-A à II-F, énumérés dans le tableau 2 :

**[Tableau 2]**

| Composé n° (anomère) | Structure |
|---|---|
| II-A (bêta, bêta) | 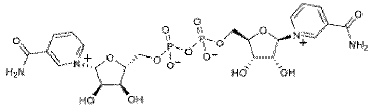 |
| II-B (bêta, alpha) | 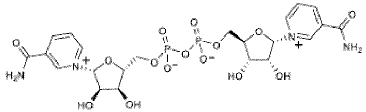 |
| II-C (alpha, alpha) | 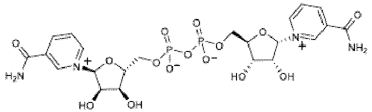 |
| II-D (beta, bêta) | 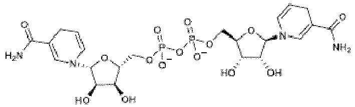 |
| II-E (bêta, alpha) | 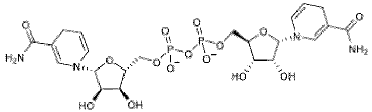 |
| II-F (alpha, alpha) | 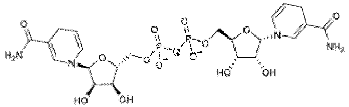 |

### FIGURES

[Fig. 1] est un graphique montrant le score nociceptif et le seuil nociceptif induit par l'administration de cyclophosphamide chez des rats par rapport aux animaux traités par le véhicule.
[Fig. 2] est un graphique montrant l'évolution du score nociceptif et du seuil nociceptif chez les rats, 2h et 4h après l'administration de cyclophosphamide par rapport au véhicule.
[Fig. 3] montre le seuil nociceptif chez les animaux traités par la cyclophosphamide auxquels a été administré le véhicule, le NMN ou l'ibuprofène à T0, T=2h ou T=4h après l'administration de cyclophosphamide.
[Fig. 4] montre le score nociceptif chez les animaux traités par la cyclophosphamide auxquels a été administré le véhicule, le NMN ou l'ibuprofène à T0, T=2h ou T=4h après l'administration de cyclophosphamide.
[Fig. 5A] est un graphique montrant le seuil nociceptif basal pour chaque groupe expérimental.
[Fig. 5B] est un graphique montrant les scores nociceptifs basaux pour tous les groupes expérimentaux.
[Fig. 6A] est un graphique montrant le seuil de nociception pour les effets du NMN, du composé A et du composé B sur l'allodynie induite par le CYP à 2h.
[Fig. 6B] est un graphique montrant le seuil de nociception pour les effets de la NMN, du composé A et du composé B sur l'allodynie induite par le CYP à 4 heures.
[Fig. 7A] est un graphique montrant les effets du NMN sur la douleur viscérale induite par le CYP à 2h (scores nociceptifs).
[Fig. 7B] est un graphique montrant les effets du NMN sur la douleur viscérale induite par le CYP à 4h (scores nociceptifs).
[Fig. 8A] est un graphique montrant les effets du composé A sur la douleur viscérale induite par le CYP à 2h (scores nociceptifs).
[Fig. 8B] est un graphique montrant les effets du composé A sur la douleur viscérale induite par le CYP à 4h (scores nociceptifs).
[Fig. 9A] est un graphique montrant les effets du composé B sur la douleur viscérale induite par le CYP à 2h (scores nociceptifs)
[Fig. 9B] est un graphique montrant les effets du composé B sur la douleur viscérale induite par le CYP à 2h (scores nociceptifs).

### EXEMPLES

Dans la suite de la présente description, les exemples sont fournis à titre illustratif de la présente invention et ne visent en aucun cas à en limiter la portée.

### Exemple 1

L'efficacité de l'utilisation de NMN selon l'invention a été évaluée chez des rats en dans un modèle de douleur nociceptive. Plus précisément, le modèle utilisé est un modèle de douleur viscérale. L'administration de cyclophosphamide (CYP) permet de simuler une cystite chez le rat.

Le contrôle positif est l'ibuprofène, un anti-inflammatoire non stéroïdien fréquemment prescrit pour soulager les douleurs nociceptives. Le contrôle négatif est le véhicule de la NMN et de l'ibuprofène, c'est-à-dire de l'eau distillée.

Pour cette étude, des rats femelles Sprague-Dawley âgées de 7 semaines ont été réparties en trois groupes, chaque groupe comprenant 6 rats :
- un groupe contrôle recevant 5 ml d'eau distillée (véhicule) ;
- un groupe traité avec du NMN à 500 mg/kg ; et
- un groupe traité avec de l'ibuprofène à 300 mg/kg.

Le NMN se trouve sous forme de zwitterion.

Après 24h d'adaptation, un test pour mesurer le seuil de tolérance à la douleur de chaque animal a été effectué avec des filaments de von Frey avant le début de l'étude. Cette mesure avant toute exposition à un stimulus douloureux permet d'obtenir le seuil de tolérance à la douleur de base chez les animaux et sert de contrôle négatif.

Les filaments de von Frey sont un dispositif permettant de mesurer la sensibilité de la peau au toucher. L'utilisation des filaments de von Frey permet de tester l'allodynie et l'hyperalgésie chez les rongeurs. Brièvement, chaque filament correspond à une force donnée. Les filaments sont appliqués dans l'ordre de force croissant sur la peau de l'animal. Les rongeurs ont en effet pour réflexe de se retirer lorsqu'un contact non attendu se produit. Le retrait en réponse à une force exercée sur celle-ci est indicatif du seuil de tolérance à la douleur de l'animal. L'utilisation des filaments de von Frey est couramment utilisée pour la mesure de la douleur chez les rongeurs (Deuis JR, Dvorakova LS and Vetter I (2017) Methods Used to Evaluate Pain Behaviors in Rodents. Front. Mol. Neurosci. 10:284.).

Dans la présente étude, huit filaments de force croissante de 1g, 2g, 4g, 6g, 8g, 10g, 15g et 26g ont été utilisés et appliqués trois fois chacun sur l'abdomen de l'animal, près de la vessie. La réponse de l'animal a été évaluée comme suit :
- score 0 : absence de réponse
- score 1 : rétractation de l'abdomen
- score 2 : piétinement ou changement de position de l'animal
- score 3 : tressaillement ou rondeur de l'abdomen ou léchage de la zone stimulée par le filament de von Frey.

Pour chaque rat, les résultats sont exprimés comme suit:
- seuil nociceptif : première force à laquelle une réponse de l'animal est observée (score de réponse supérieur ou égal à 1). Cela indique le seuil de tolérance à la douleur le plus bas pour mesurer l'allodynie ;
- score nociceptif : pourcentage de la réponse maximale pour chaque filament. Cela indique la réponse à la douleur globale.

Les animaux de chacun des groupes reçoivent soit le véhicule, soit le NMN à 500 mg/kg, soit l'ibuprofène à 300 mg/kg par voie orale. Après administration des composés à tester (véhicule, NMN ou ibuprofène), la cyclophosphamide est injectée par voie intrapéritonéale chez chaque rat. La cyclophosphamide induit une forte inflammation au niveau de la vessie et simule une douleur induite par une infection urinaire comme la cystite.

Le test avec les filaments de von Frey est réitéré à 2h puis à 4h après injection de la cyclophosphamide pour mesurer la réponse à la douleur des animaux.

Les résultats ont été analysés par un test ANOVA à un facteur complété par un test de Dunnett ou par une analyse ANOVA à deux facteurs. Concernant la significativité statistique, * signifie que p<0.05, ** signifie que p<0.01 et *** signifie que p<0.001, en comparaison avec le groupe traité par le véhicule.

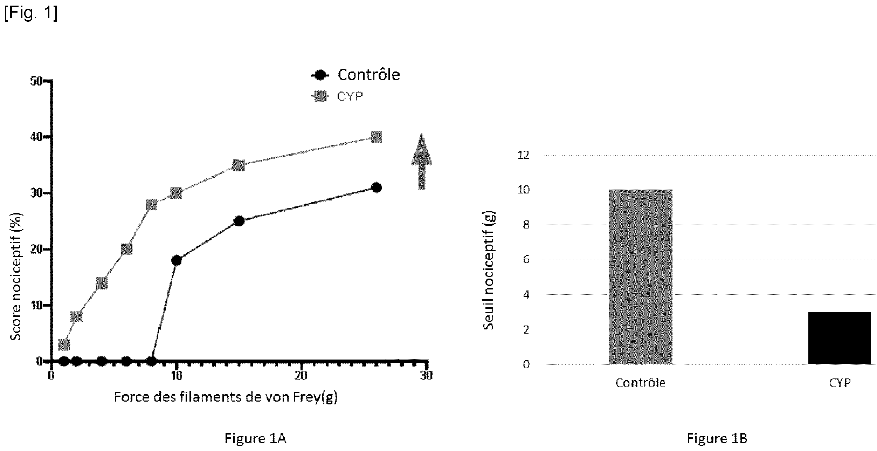

Comme on peut le voir sur la figure 1A, l'administration de cyclophosphamide déclenche une réponse des rats dès l'application du premier filament de 1g tandis que la réponse des animaux avant exposition montre que les animaux ne présentent aucune réponse à la douleur jusqu'à 10 g avant administration de la cyclophosphamide : l'injection de cyclophosphamide abaisse donc le seuil d'allodynie. Ce résultat est corroboré par la figure 1B qui montre que l'administration de cyclophosphamide réduit le seuil de tolérance à la douleur de 10g à 3g. En d'autres termes, le seuil d'allodynie est considérablement réduit par l'injection de cyclophosphamide chez les rats. Les animaux traités par la cyclophosphamide sont donc plus sensibles à la douleur.

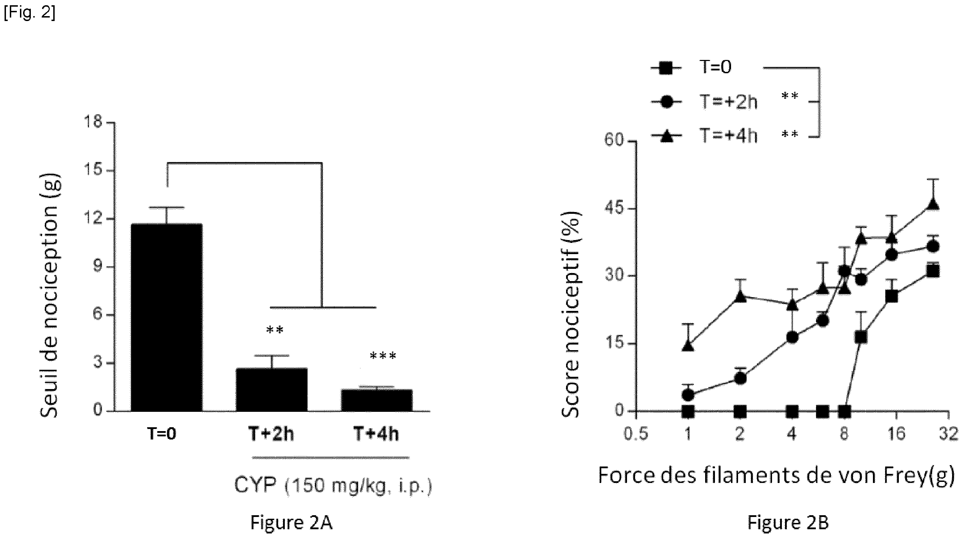

Les figures 2A et 2B montrent que la réduction du seuil d'allodynie se poursuit, les animaux montrant encore moins de tolérance à la douleur 4h après l'injection de cyclophosphamide par rapport à 2h après injection.

La figure 1A montre en outre qu'à force égale, les animaux ressentent davantage de douleur : l'injection de cyclophosphamide déclenche donc une hyperalgésie. La figure 2B montre que cet effet se poursuit avec le temps, les scores de douleur mesurés après 4h chez les rats traités étant plus élevés que la mesure effectuée 2h après l'injection.

L'injection de cyclophosphamide induit donc d'une part une réduction de l'allodynie et une augmentation de l'hyperalgésie chez les animaux traités, les effets étant plus accentués avec le temps.

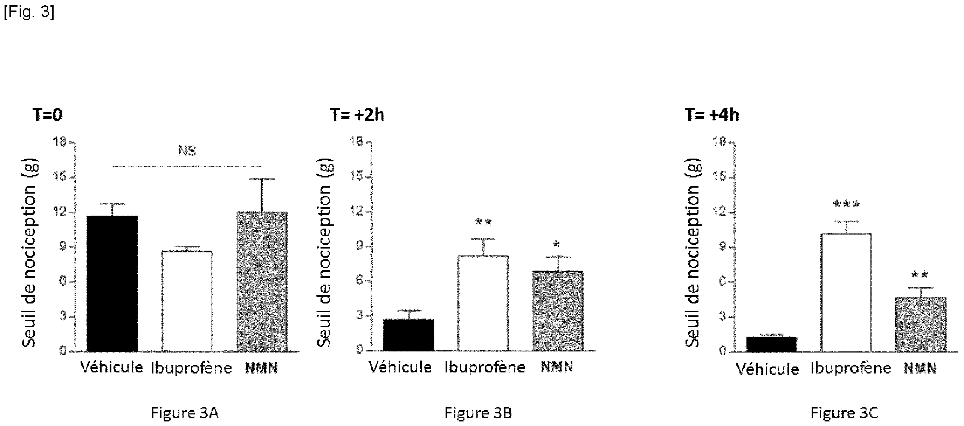

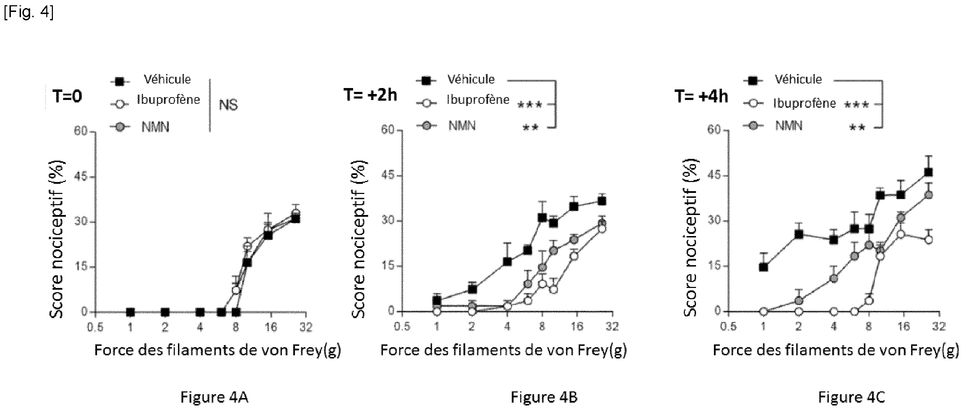

Les figures 3 et 4 montrent les effets de l'administration de NMN et d'ibuprofène sur les seuils et les scores de douleurs chez les rats traités par la cyclophosphamide.

Comme le montrent les figures 3A, 3B et 3C, l'administration de NMN permet d'augmenter le seuil de réponse à la douleur nociceptive de manière significative, à T0, T=2h après injection et T=4h après injection. De manière attendue, l'ibuprofène permet également d'augmenter de manière significative le seuil de tolérance à la douleur chez les rats traités par la cyclophosphamide à T0, T=2h après injection et T=4h après injection. Ces résultats montrent que l'administration de NMN permet de réduire l'allodynie.

Comme le montrent la figure 4A, le NMN, l'ibuprofène et le véhicule montrent des courbes de scores nociceptives similaires à T0, au moment de l'injection de cyclophosphamide. Ceci démontre que les animaux ne développent pas de douleur en réponse à l'administration de NMN ou d'ibuprofène. En revanche, les figures 4B et 4C montrent que l'administration de NMN permet de réduire le score nociceptif 2h et 4h après l'administration de cyclophosphamide, de même que l'ibuprofène.

L'administration de NMN et de composition le comprenant permet donc de réduire l'allodynie et l'hyperalgésie de manière significative, et de manière similaire à l'ibuprofène.

### Exemple 2

### Synthèse du composé de l'invention

### Matériel et méthodes

Tous les réactifs ont été obtenus auprès de fournisseurs commerciaux et utilisés sans autre purification. La chromatographie sur couche mince a été réalisée sur des feuilles de plastique CCM de gel de silice 60F254 (épaisseur de la couche 0,2 mm) de Merck. La purification par chromatographie sur colonne a été effectuée sur gel de silice 60 (70-230 mesh ASTM, Merck). Les points de fusion ont été déterminés soit sur un appareil numérique (Electrothermal IA 8103) et ne sont pas corrigés, soit sur un banc Kofler de type WME (Wagner & Munz). Les spectres RMN IR, ¹H, ¹⁹F et ¹³C ont confirmé les structures de tous les composés. Les spectres IR ont été enregistrés sur un spectromètre FT-IR Perkin Elmer Spectrum 100 et les spectres RMN ont été enregistrés, en utilisant CDCl₃, CD₃CN, D₂O ou DMSO-d₆ comme solvant, sur un spectromètre BRUKER AC 300 ou 400 à 300 ou 400 MHz pour les spectres ¹H, 75 ou 100 MHz pour le ¹³C et 282 ou 377 MHz pour le ¹⁹F. Les décalages chimiques (δ) ont été exprimés en parties par million par rapport au signal, indirectement (i) au CHCl₃ (δ 7.27) pour ¹H et (ii) au CDCl₃ (δ 77.2) pour ¹³C et directement (iii) au CFCl₃ (étalon interne) (δ 0) pour ¹⁹F. Les déplacements chimiques sont donnés en ppm et les multiplicités de pics sont désignées comme suit : s, singulet ; br s, singulet large ; d, doublet ; dd, doublet de doublet ; t, triplet ; q, quadruplet ; quint, quintuplet ; m, multiplet. Les spectres de masse à haute résolution (HRMS) ont été obtenus auprès du "Service central d'analyse de Solaize" (Centre national de la recherche scientifique) et ont été enregistrés sur un spectromètre Waters utilisant l'ionisation par électrospray-TOF (ESI-TOF).

### Protocole

### Etape 1 : Synthèse du composé de formule X-1

Le composé de formule XIV (1,0 équiv.) est dissous dans le dichlorométhane. Le nicotinamide de formule XV (1,50 équiv.) et le TMSOTf (1,55 équiv.) sont ajoutés à température ambiante. Le mélange réactionnel est chauffé au reflux et agité jusqu'à l'achèvement. Le mélange est refroidi à température ambiante et filtré. Le filtrat est concentré à sec pour donner le tétraacétate de NR brut de formule X-1.

### Étape 2 : Synthèse du composé de formule X

Le tétraacétate de NR brut de formule X-1 est dissous dans du méthanol et refroidi à -10 °C. On ajoute de l'ammoniac 4,6 M dans le méthanol (3,0 équivalents) à -10 °C et on agite le mélange à cette température jusqu'à achèvement. Du Dowex HCR (H⁺) est ajouté jusqu'à ce que le pH soit de 6-7. Le mélange réactionnel est chauffé à 0°C et filtré. La résine est lavée avec un mélange de méthanol et d'acétonitrile. Le filtrat est concentré jusqu'à ce qu'il soit sec. Le résidu est dissous dans l'acétonitrile et concentré jusqu'à siccité. Le résidu est dissous dans l'acétonitrile pour donner une solution de triflate de NR brut de formule X.

### Étape 3 : Synthèse du composé de formule XI

La solution de triflate de NR brut dans l'acétonitrile est diluée avec du phosphate de triméthyle (10,0 équivalents). L'acétonitrile est distillé sous vide et le mélange est refroidi à -10 °C. L'oxychlorure de phosphore (4,0 équiv.) est ajouté à -10 °C et le mélange est agité à -10 °C jusqu'à l'achèvement.

### Etape 4 et étape 5 : Synthèse du composé de formule I-A (non revendiqué)

Le mélange est hydrolysé par addition d'un mélange 50/50 d'acétonitrile et d'eau, suivi de l'addition d'éther méthylique de tert-butyle. Le mélange est filtré et le solide est dissous dans l'eau. La solution aqueuse est neutralisée par addition de bicarbonate de sodium et extraite avec du dichlorométhane. La couche aqueuse est concentrée à sec pour donner un mélange brut de NMN et de di-NMN de formule I-A.

### Isolement du di-NMN de formule I-A :

Le NMN et le di-NMN de formule I-A sont séparés par purification sur Dowex 50wx8 avec élution d'eau. Les fractions contenant du di-NMN sont concentrées jusqu'à siccité. Le résidu est purifié par chromatographie sur colonne sur gel de silice (gradient isopropanol/eau). Les fractions pures sont combinées et concentrées. Le résidu est lyophilisé pour donner du di-NMN sous forme de solide beige.

### Données biologiques

L'objectif de la présente étude est d'évaluer les effets de l'administration orale de mononucléotide de nicotinamide (NMN), d'alpha-NMN (composé A) et du composé I-A (composé B, non-revendiqué) à 500 mg/kg sur la réponse de la douleur viscérale dans le modèle de cystite aiguë induite par le cyclophosphamide (CYP) chez des rats Sprague-Dawley femelles.

### Matériels et méthodes

Animaux : Rats femelles Sprague-Dawley, 7 semaines à la naissance
Traitement pharmacologique :
   - NMN : 500 mg/kg
   - alpha -NMN : 500 mg/kg
   - composé I-A: 500 mg/kg
   - Véhicule : eau distillée
   - Voie d'administration : per os (p.o.), 5 ml/kg
   - Fréquence d'administration : une fois à J0, 15 min avant l'injection intrapéritonéale (i.p.) de CYP.
*Cystite aiguë induite par le CYP :* Le CYP a été injecté par voie i.p. à 150 mg/kg dans un volume final de 5 ml/kg de solution saline.

### Stimulation mécanique à l'aide de filaments de Von Frey

Les rats ont été placés dans des boîtes individuelles en plexiglas avec un sol en grillage métallique et ont pu s'adapter à la chambre pendant au moins 30 minutes avant le début de tout test. Huit filaments de von Frey avec des forces croissantes de 1, 2, 4, 6, 8, 10, 15 et 26 g ont été utilisés. Chaque filament calibré a été appliqué 3 fois dans la zone abdominale inférieure, près de la vessie.

### Evaluation des comportements nociceptifs pour chaque application

- Score 0 = aucune réponse
- Score 1 = rétraction de l'abdomen
- Score 2 = piétinement ou changement de poste
- Score 3 = sifflement ou courbure abdominale ou léchage du site stimulé avec des filaments de von Frey

Pour chaque rat, les résultats ont été exprimés comme suit :
- Seuil nociceptif : première force de von Frey pour laquelle le stimulus est perçu comme douloureux (le score ≥ 1 est obtenu) => seuil abaissé = allodynie
- Score nociceptif : % de la réponse maximale (total = 9 pour 3 applications regroupées) pour chaque filament => Réponse globale à la douleur

Groupes expérimentaux (6 rats par groupe):
- Groupe 1 : Véhicule (5 ml/kg) + CYP
- Groupe 2 : NMN (500 mg/kg) + CYP
- Groupe 3 : Composé A (500 mg/kg) + CYP
- Groupe 4: Composé B (500 mg/kg) + CYP

### Résultats

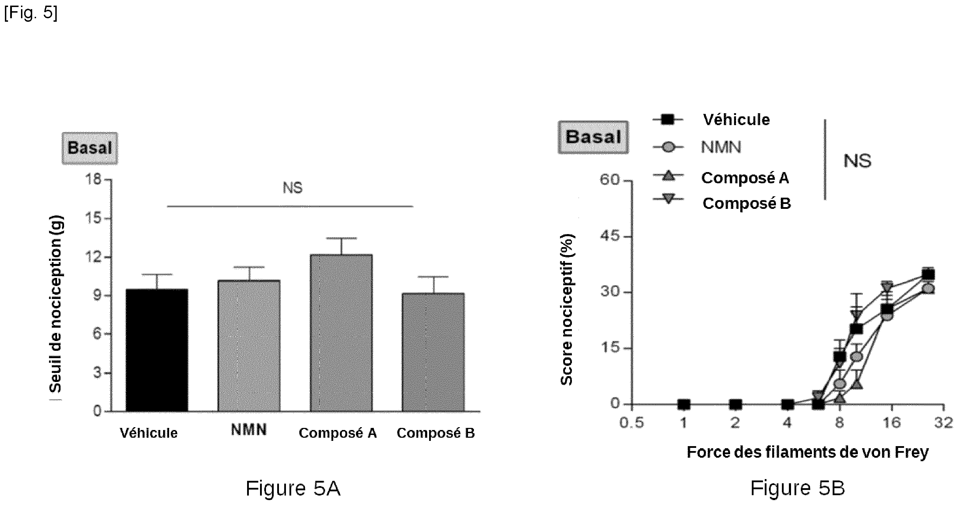

Paramètres nociceptifs de base (avant l'injection de CYP) pour tous les groupes expérimentaux : Les résultats montrent (figures 5A et 5B) que les réponses nociceptives basales étaient similaires entre tous les groupes expérimentaux (avant l'injection de CYP).

Douleur viscérale induite par le CYP 2h et 4h après l'injection (en comparaison avec la valeur basale dans le groupe du véhicule) : Les résultats montrent que par rapport à la réponse basale, le CYP (150 mg/kg, i.p.) a induit une diminution significative du seuil nociceptif (figure 2A) et une augmentation significative des scores nociceptifs (figure 2B) aux deux points temporels.

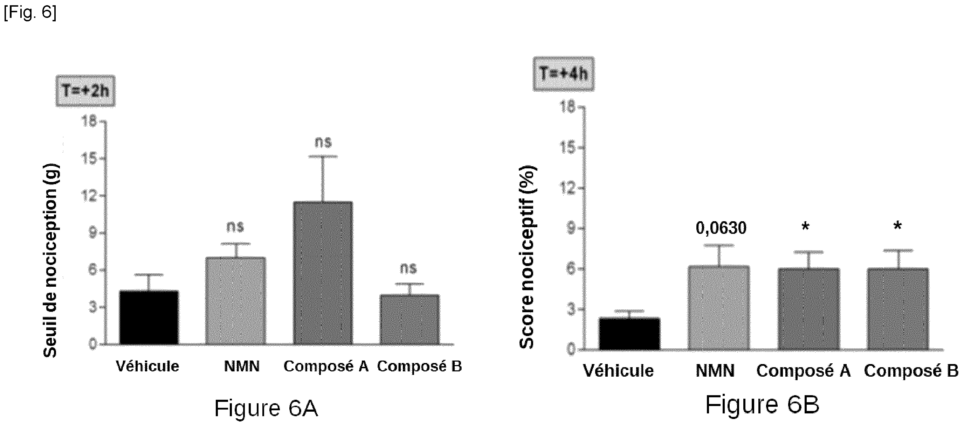

Effet du NMN, du composé A et du composé B sur l'allodynie induite par le CYP (seuil nociceptif) : Les résultats montrent que, par rapport au véhicule :
- le NMN (500 mg/kg, p.o) a entraîné une légère augmentation du seuil nociceptif à +2h (figure 6A) et +4h (figure 6B) avec un effet juste au-dessus de la marge de signification statistique à +4h (p=0,063),
- Le composé A (500mg/kg, p.o) a entraîné une augmentation du seuil nociceptif à +2 (figure 6A) et +4h (significatif à +4h) (figure 6B),
- Le composé B (500mg/kg, p.o) a entraîné une augmentation significative du seuil nociceptif seulement à +4h (figure 6B) et aucun effet à +2h (figure 6A).

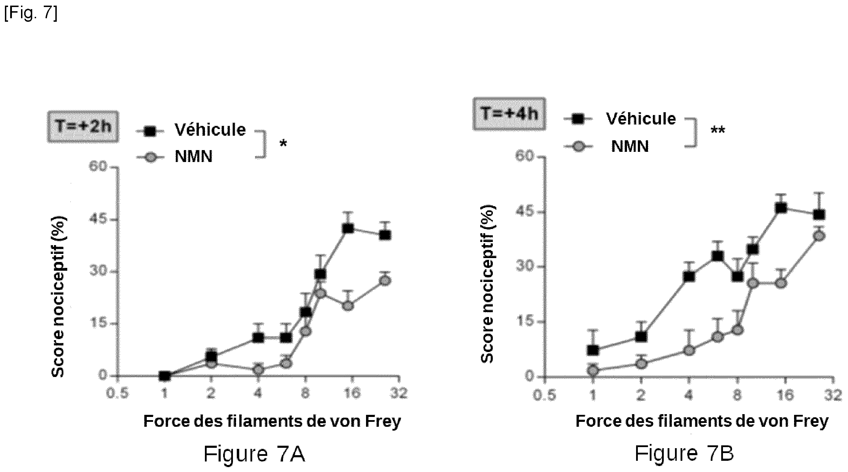

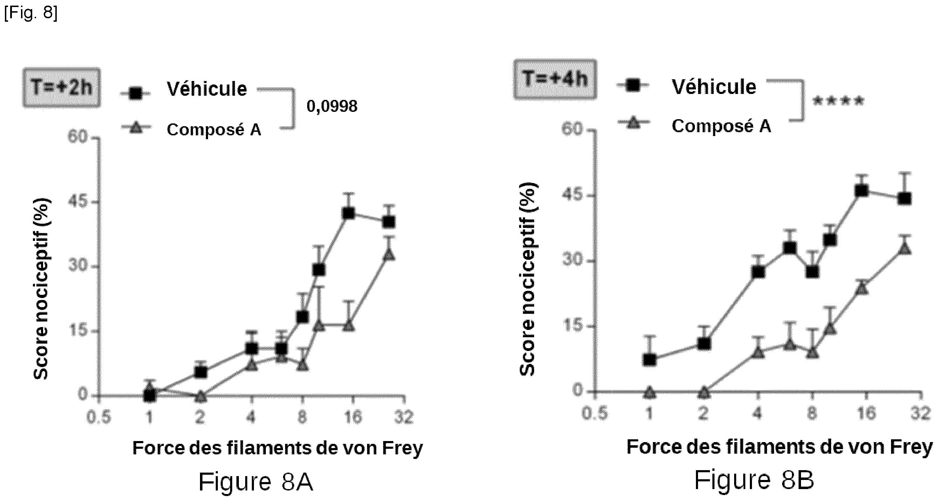

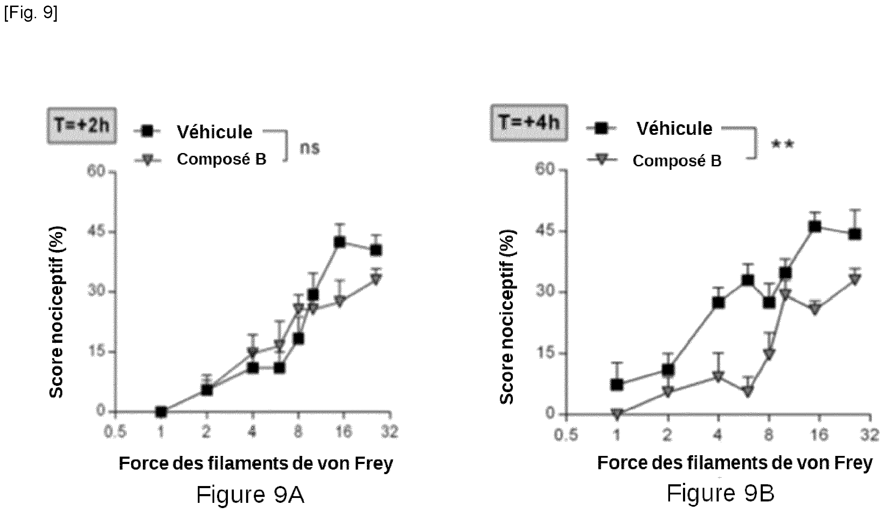

Effets du NMN, du composé A et du composé B sur la douleur viscérale induite par le CYP (scores nociceptifs) : Les résultats montrent (figures 7, 8 et 9) que par rapport au véhicule :
- NMN (500 mg/kg, p.o) a conduit à une diminution significative des scores nociceptifs à +2 (figure 7A) et +4h (figure 7B),
- Le composé A (500mg/kg, p.o) a entraîné une diminution des scores nociceptifs à +2 (figure 8A) et +4h (figure 8B) qui n'ont atteint le niveau statistique qu'à +4h,
- Le composé B (500mg/kg, p.o) a entraîné une diminution significative des scores nociceptifs à +4h (figure 9B) (aucun effet n'a été observé à +2h (figure 9A)).

### Résumé des résultats

Les réponses nociceptives basales étaient similaires dans tous les groupes expérimentaux (avant l'injection de CYP).

En comparaison avec la réponse basale, les effets du CYP (150 mg/kg, i.p.) à 2 et 4 heures ont été caractérisés par :
- Une diminution significative du seuil nociceptif à +2 et +4h,
- Une augmentation significative des scores nociceptifs à +2 et +4h.

Par rapport au véhicule, chez les rats injectés de CYP, les effets de la NMN (500 mg/kg, p.o.) ont conduit à :
- une légère augmentation du seuil nociceptif à +2 et +4h avec un effet juste au-dessus de la marge de signification statistique à +4h (p=0.063)
- une diminution significative des scores nociceptifs à +2 et +4h.

Par rapport au véhicule, chez les rats ayant reçu une injection de CYP, les effets du composé A (500 mg/kg, p.o.) ont été caractérisés par :
- Une augmentation du seuil nociceptif à +2 et +4h avec un effet qui n'a atteint le niveau de signification qu'à +4h,
- Une diminution des scores nociceptifs à +2 et +4h qui n'ont atteint le niveau statistique qu'à +4h.

Par rapport au véhicule, chez les rats ayant reçu une injection de CYP, les effets du composé B (500 mg/kg, p.o.) ont entraîné :
- Une augmentation significative du seuil nociceptif à +4h (aucun effet n'a été observé à +2h),
- Une diminution significative des scores nociceptifs à +4h (aucun effet n'a été observé à +2h).

### Conclusion

Une seule injection intrapéritonéale de CYP (150 mg/kg) a induit une douleur viscérale 2 et 4 heures après l'injection avec un effet plus prononcé à +4h.

Un traitement oral unique de NMN (500 mg/kg) a soulagé la douleur viscérale induite par le CYP aux deux moments, avec un niveau de signification plus élevé obtenu à +4h.

L'administration d'alpha-NMN (composé A) a réduit la douleur viscérale induite par le CYP à +2 et +4h mais ses effets n'ont atteint la significativité qu'à +4h.

Chez les rats injectés de CYP, le traitement oral avec composé I-A (composé B) n'a eu aucun effet bénéfique à +2h mais a montré une activité anti-nociceptive significative à un moment ultérieur (c'est-à-dire +4h).

Les inventeurs ont donc démontré que l'utilisation de NMN et de ses dérivés pharmaceutiquement acceptables, tels que l'alpha NMN et le composé I-A, ainsi que de compositions les comprenant conformément à l'invention, permettait de réduire la douleur nociceptive, et plus particulièrement la douleur viscérale induite par la cystite.

L'utilisation de NMN et de ses dérivés pharmaceutiquement acceptables, tels que l'alpha NMN et le composé I-A, ainsi que de compositions les comprenant conformément à l'invention permet donc de traiter et prévenir la douleur, en particulier la douleur nociceptive.

## Revendications

1. Nicotinamide mononucléotide (NMN), un de ses dérivés pharmaceutiquement acceptables ou un de ses sels pharmaceutiquement acceptables, le dérivé du NMN étant choisi parmi le dihydronicotinamide mononucléotide (noté NMN-H), un composé de formule (I) ou (II) ou un de ses stéréoisomères, un de ses sels, un de ses hydrates, un de ses solvates ou un de ses cristaux, ledit composé de formule (I) étant choisi parmi :
| **Composés (anomères)** | **Structure** |
|---|---|
| I-C (beta) | 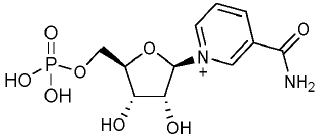 |
| I-D (alpha) | 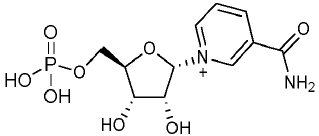 |
| I-G (beta) | 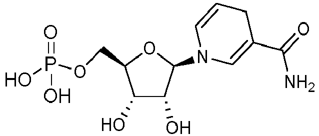 |
| I-H (alpha) | 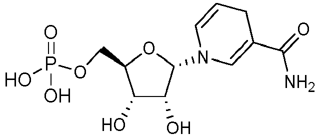 |
| I-J (beta) | 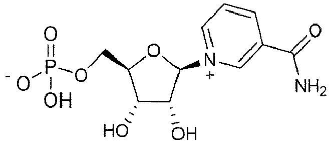 |
| I-K (alpha) | 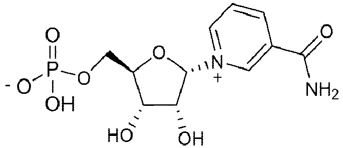 |
ou
ledit composé de formule (II) étant choisi parmi :
| Composé n° (anomère) | Structure |
|---|---|
| II-A (bêta, bêta) | 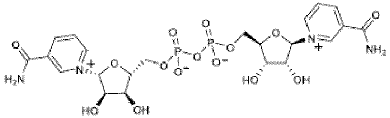 |
| II-B (bêta, alpha) | 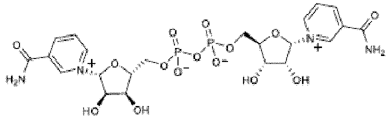 |
| II-C (alpha, alpha) | 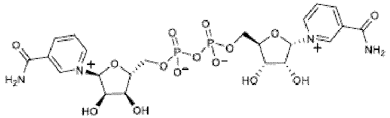 |
| II-D (bêta, bêta) | 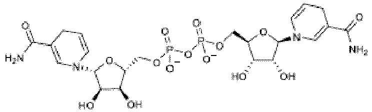 |
| II-E (bêta, alpha) | 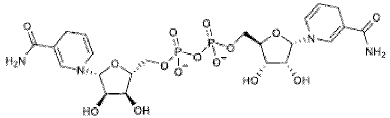 |
| II-F (alpha, alpha) | 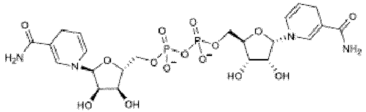 |
et leurs combinaisons,
pour son utilisation dans la prévention et/ou le traitement de la douleur dans laquelle la douleur est une douleur nociceptive viscérale et n'est pas une douleur neuropathique.

2. Nicotinamide mononucléotide, un de ses dérivés pharmaceutiquement acceptables ou un de ses sels pharmaceutiquement acceptables, pour son utilisation selon la revendication 1 pour réduire l'allodynie.

3. Nicotinamide mononucléotide, un de ses dérivés pharmaceutiquement acceptables ou un de ses sels pharmaceutiquement acceptables, pour son utilisation selon l'une des revendications précédentes pour réduire l'hyperalgésie.

4. Nicotinamide mononucléotide, un de ses dérivés pharmaceutiquement acceptables ou un de ses sels pharmaceutiquement acceptables, pour son utilisation selon l'une des revendications précédentes dans laquelle la douleur est une douleur causée par une infection urinaire.

5. Nicotinamide mononucléotide, un de ses dérivés pharmaceutiquement acceptables ou un de ses sels pharmaceutiquement acceptables, pour son utilisation selon l'une des revendications précédentes en combinaison avec au moins un autre agent thérapeutique.

6. Nicotinamide mononucléotide, un de ses dérivés pharmaceutiquement acceptables ou un de ses sels pharmaceutiquement acceptables pour son utilisation selon la revendication 5 dans laquelle l'au moins un agent thérapeutique est choisi parmi les antibiotiques, les antifongiques, les antiviraux et leurs combinaisons.

7. Nicotinamide mononucléotide, un de ses dérivés pharmaceutiquement acceptables ou un de ses sels pharmaceutiquement acceptables pour son utilisation selon la revendication 5 ou 6 dans laquelle l'au moins un agent thérapeutique est un antalgique.

8. Composition comprenant du nicotinamide mononucléotide, un de ses dérivés pharmaceutiquement acceptables ou un de ses sels pharmaceutiquement acceptables et au moins un excipient pharmaceutiquement acceptable pour son utilisation selon l'une des revendications 1 à 7.

9. Composition pour son utilisation selon la revendication 8 comprenant en outre au moins un agent thérapeutique supplémentaire.

## Patentansprüche

1. Nicotinamidmononukleotid (NMN), eines seiner pharmazeutisch annehmbaren Derivate oder eines seiner pharmazeutisch annehmbaren Salze, wobei das NMN-Derivat ausgewählt ist aus Dihydronicotinamidmononukleotid (bezeichnet als NMN-H), einer Verbindung der Formel (1) oder (II) oder einem Stereoisomer davon, einem Salz davon, einem Hydrat davon, einem Solvat davon oder einem Kristall davon, wobei die Verbindung von Formel (1) ausgewählt ist aus:
| **Verbindung (Anomere)** | **Struktur** |
|---|---|
| I-C (beta) | 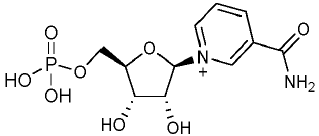 |
| I-D (alpha) | 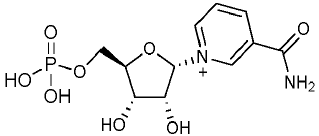 |
| I-G (beta) | 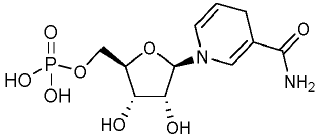 |
| I-H (alpha) | 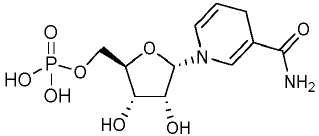 |
| I-J (beta) | 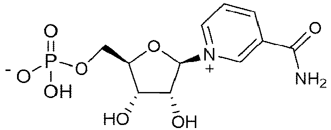 |
| I-K (alpha) | 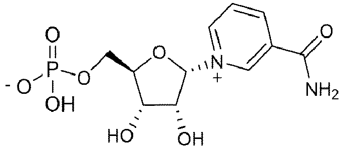 |
oder
wobei die Verbindung von Formel (II) ausgewählt ist aus:
| Verbindung n°(Anomer) | Struktur |
|---|---|
| II-A (beta, beta) | 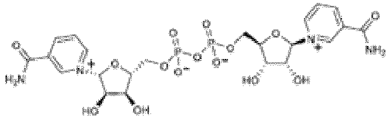 |
| II-B (beta, alpha) | 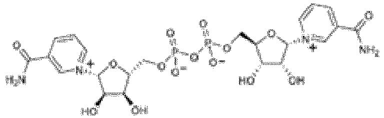 |
| II-C (alpha, alpha) | 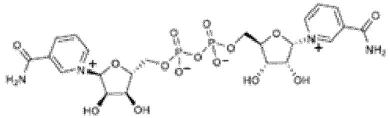 |
| II-D (beta, beta) | 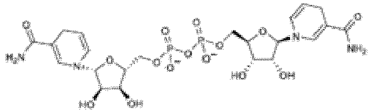 |
| II-E (beta, alpha) | 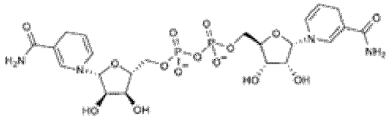 |
| II-F (alpha, alpha) | 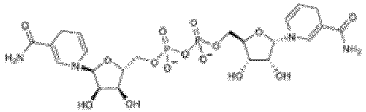 |
und ihren Kombinationen,
zur Verwendung bei der Prävention und/oder Behandlung von Schmerzen, bei denen der Schmerz ein viszeraler nozizeptiver Schmerz ist und kein neuropathischer Schmerz.

2. Nicotinamidmononukleotid, eines seiner pharmazeutisch annehmbaren Derivate oder eines seiner pharmazeutisch annehmbaren Salze zur Verwendung nach Anspruch 1 zur Verringerung von Allodynie.

3. Nicotinamidmononukleotid, eines seiner pharmazeutisch annehmbaren Derivate oder eines seiner pharmazeutisch annehmbaren Salze zur Verwendung nach einem der vorherigen Ansprüche zur Verringerung von Hyperalgesie.

4. Nicotinamidmononukleotid, ein pharmazeutisch annehmbares Derivat davon oder ein pharmazeutisch annehmbares Salz davon, zur Verwendung nach einem der vorherigen Ansprüche, wobei der Schmerz ein durch eine Harnwegsinfektion verursachter Schmerz ist.

5. Nicotinamidmononukleotid, eines seiner pharmazeutisch verträglichen Derivate oder eines seiner pharmazeutisch verträglichen Salze zur Verwendung nach einem der vorherigen Ansprüche in Kombination mit mindestens einem anderen therapeutischen Mittel.

6. Nicotinamidmononukleotid, eines seiner pharmazeutisch annehmbaren Derivate oder eines seiner pharmazeutisch annehmbaren Salze zur Verwendung nach Anspruch 5, wobei das mindestens eine therapeutische Mittel ausgewählt ist aus Antibiotika, Antimykotika, antiviralen Mitteln und Kombinationen davon.

7. Nicotinamidmononukleotid, eines seiner pharmazeutisch annehmbaren Derivate oder eines seiner pharmazeutisch annehmbaren Salze zur Verwendung nach Anspruch 5 oder 6, wobei das mindestens eine therapeutische Mittel ein Analgetikum ist.

8. Zusammensetzung, umfassend Nicotinamidmononukleotid, ein seiner pharmazeutisch annehmbaren Derivate oder ein seiner pharmazeutisch annehmbaren Salze zur Verwendung und mindestens einen pharmazeutisch annehmbaren Hilfsstoff zur Verwendung nach einem der Ansprüche 1 bis 7.

9. Zusammensetzung zur Verwendung nach Anspruch 8, ferner umfassend mindestens ein weiteres therapeutisches Mittel.

## Claims

1. Nicotinamide mononucleotide (NMN), a pharmaceutically acceptable derivative thereof or a pharmaceutically acceptable salt thereof, the derivative of NMN being selected from among dihydronicotinamide mononucleotide (denoted NMN-H), a compound of formula (I) or (II), a stereoisomer thereof, a salt thereof, a hydrate thereof, a solvate thereof or a crystal thereof, said compound of formula (I) being selected from among:
| **Compounds (anomers)** | **Structure** |
|---|---|
| I-C (beta) | 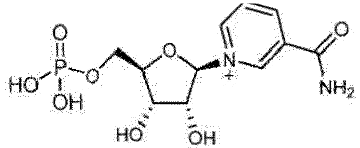 |
| I-D (alpha) | 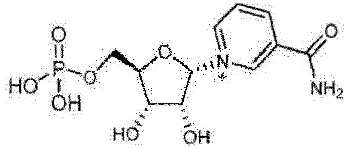 |
| I-G (beta) | 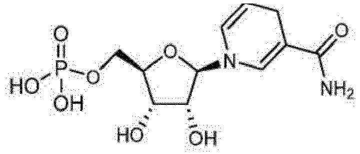 |
| I-H (alpha) | 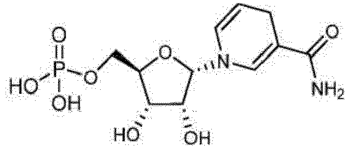 |
| I-J (beta) | 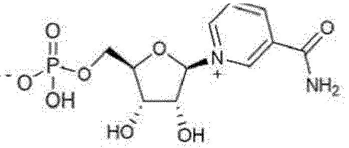 |
| I-K (alpha) | 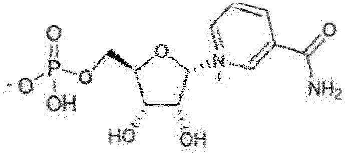 |
or:
said compound of formula (II) being selected from among:
| **Compound N° (anomer)** | **Structure** |
|---|---|
| II-A (beta, beta) | 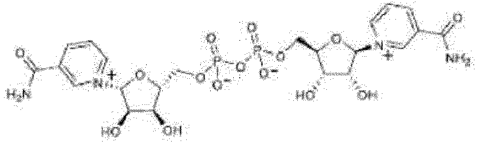 |
| II-B (beta, alpha) | 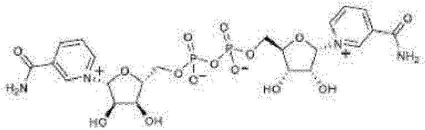 |
| II-C (alpha, alpha) | 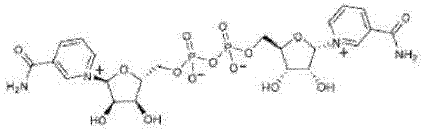 |
| II-D (beta, beta) | 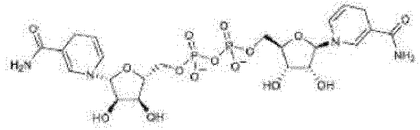 |
| II-E (beta, alpha) | 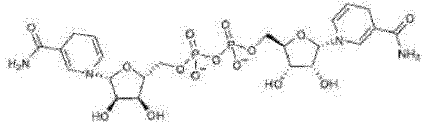 |
| II-F (alpha, alpha) | 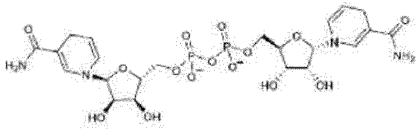 |
and combinations thereof,
for use thereof in the prevention and/or treatment of pain, wherein the pain is a visceral nociceptive pain and is not a neuropathic pain.

2. Nicotinamide mononucleotide, a pharmaceutically acceptable derivative thereof or a pharmaceutically acceptable salt thereof, for use according to claim 1 to reduce allodynia.

3. Nicotinamide mononucleotide, a pharmaceutically acceptable derivative thereof or a pharmaceutically acceptable salt thereof, for use according to any of the preceding claims to reduce hyperalgesia.

4. Nicotinamide mononucleotide, a pharmaceutically acceptable derivative thereof or a pharmaceutically acceptable salt thereof, for use according to any of the preceding claims, wherein the pain is pain caused by a urinary infection.

5. Nicotinamide mononucleotide, a pharmaceutically acceptable derivative thereof or a pharmaceutically acceptable salt thereof, for use according to any of the preceding claims in combination with at least one other therapeutic agent.

6. Nicotinamide mononucleotide, a pharmaceutically acceptable derivative thereof or a pharmaceutically acceptable salt thereof, for use according to claim 5, wherein the at least one therapeutic agent is selected from among antibiotics, antifungals, antivirals and combinations thereof.

7. Nicotinamide mononucleotide, a pharmaceutically acceptable derivative thereof or a pharmaceutically acceptable salt thereof, for use according to claim 5 or 6, wherein the at least one therapeutic agent is an analgesic.

8. A composition comprising nicotinamide mononucleotide, a pharmaceutically acceptable derivative thereof or a pharmaceutically acceptable salt thereof and at least one pharmaceutically acceptable excipient for use according to any of claims 1 to 7.

9. A composition for use thereof according to claim 8, further comprising at least one additional therapeutic agent.
